# EUROPÄISCHE PATENTSCHRIFT

(11) **EP 3 206 732 B1**
(45) Veröffentlichungstag und Bekanntmachung des Hinweises auf die Patenterteilung: **19.04.2023**
(21) Anmeldenummer: 15775473.0
(22) Anmeldetag: 09.10.2015
(51) Int. Cl.: A61M 1/16, A61M 1/34, A61M 1/36

(54) **VORRICHTUNG ZUR EREIGNISABHÄNGIGEN GRENZWERTÜBERWACHUNG EINER DIALYSEMASCHINE**
DEVICE FOR EVENT-DEPENDENT BOUNDARY MONITORING IN A DIALYSIS MACHINE
DISPOSITIF DE SURVEILLANCE DE LA VALEUR LIMITE D'UNE MACHINE DE DIALYSE EN FONCTION D'ÉVÉNEMENTS

(30) Priorität: 15.10.2014 DE 102014015048
(43) Veröffentlichungstag der Anmeldung: 23.08.2017
(73) Patentinhaber: Fresenius Medical Care Deutschland GmbH, 61352 Bad Homburg (DE)
(72) Erfinder: BREUEL, Lars, 99096 Erfurt (DE); MERTEN, Kai, 97537 Wipfeld (DE); VERCH, Georg, 65191 Wiesbaden (DE); HAULSEN, Jan, 64560 Riedstadt (DE)
(74) Vertreter: Schwarz, Claudia
(86) Internationale Anmeldenummer: PCT/EP2015/073458
(87) Internationale Veröffentlichungsnummer: WO 2016/058954

(56) Entgegenhaltungen:
- WO-A2-2014/144909

## Beschreibung

Die Erfindung betrifft ein Verfahren zur ereignisgesteuerten Grenzwertsetzung im Rahmen einer Geräteüberwachung auf Einhaltung von Grenzwerten in einem medizinischen Flüssigkeitskreislaufsystem, insbesondere einem extrakorporalen Blutkreislaufsystem und umfasst auch eine Vorrichtung dafür. Sie eignet sich besonders für einen extrakorporalen Dialysekreislauf.

Der extrakorporale Blutkreislauf ist im Allgemeinen mit einer Überwachungseinheit ausgestattet, die permanent den arteriellen und venösen Druck innerhalb des Kreislaufs überwacht. Zweck der Drucküberwachung ist die Erkennung von verschiedenen Komplikationen, die während der extrakorporalen Blutbehandlung auftreten können. Zu den möglichen Komplikationen zählt ein fehlerhafter Gefäßzugang, der beispielsweise auf das Herausrutschen oder Ansaugen der Kanüle zurück zu führen ist. Eine Druckveränderung tritt auch bei einer Veränderung des von einer Blutpumpe geförderten Blutvolumens ein.

Bei den bekannten Überwachungseinheiten ist es vorgesehen, dass bei Nichteinhaltung von vorbestimmten, festen Grenzwerten, ein Alarm ausgelöst wird, um die Patientensicherheit ständig und vollständig gewährleisten zu können.

Insbesondere während des Betriebs gibt es jedoch Ereignisse, die eine dynamische Anpassung bzw. Veränderung der Grenzwerte oder Grenzwertbereiche erfordern. Bei den bekannten Überwachungssystemen werden diese Grenzwerte in der Regel nicht dynamisch gesetzt, sondern sie sind statisch vorbestimmt. Dadurch kann es in bestimmten Situationen zur Auslösung von Fehlalarmen kommen, die sich nachteilig auf die Behandlung auswirken. So kann es nachteiliger Weise zu einer zeitlichen Verzögerung kommen, da unter Umständen Maßnahmen eingeleitet werden, die nicht notwendig sind und/oder die ihrerseits wiederum Auswirkungen auf andere Grenzwertüberwachungen haben können.

Ausgehend von diesem Stand der Technik hat sich die vorliegende Erfindung zur Aufgabe gestellt, ein Verfahren und eine Vorrichtung und damit eine Lösung zu schaffen, um eine Vielzahl von grenzwertrelevanten Ereignissen bei der Grenzwertsetzung im Rahmen einer Geräteüberprüfung zu berücksichtigen. Dabei sollen die Grenzwerte auch dynamisch an aktuelle Systemzustände angepasst werden können. Des Weiteren sollen Querabhängigkeiten zwischen den grenzwertrelevanten Ereignissen berücksichtigt werden können ohne, dass komplexe Abfragelogiken implementiert werden müssen.

Diese Aufgabe wird durch die nebengeordneten beiliegenden Patentansprüche gelöst, insbesondere durch ein Verfahren und durch eine Grenzwertsetzvorrichtung oder eine Überwachungseinrichtung mit einer Grenzwertsetzvorrichtung.

Die Grenzwertsetzvorrichtung ist gemäß einem Aspekt der Erfindung ausgebildet, um alle Schritte des Verfahrens auszuführen. Dabei können die funktionalen Merkmale des Verfahrensanspruchs zumindest teilweise durch entsprechende Mikroprozessormodule implementiert sein (wie z.B. das funktionale Merkmal Vergleich durch eine Komparatorschaltung oder das funktionale Merkmal der Steuerung durch ein Steuermodul oder eine Überwachungseinheit).

Gemäß einem Aspekt betrifft die Erfindung ein Verfahren zur ereignisgesteuerten Grenzwertsetzung eines medizinischen Gerätes, insbesondere eines Hämodialysegerätes, im Rahmen einer Geräteüberwachung auf Einhaltung von Grenzwerten in einem medizinischen Flüssigkeitskreislaufsystems. Das Verfahren umfasst folgende Verfahrensschritte:
- Vorbestimmen einer Vielzahl von grenzwertrelevanten Ereignissen, die bei der Geräteüberwachung zu berücksichtigen sind und die in einer Zustandstabelle gespeichert werden. Die Ereignisse können insbesondere jeweils als Zustandsvektoren in der Zustandstabelle gespeichert werden.
- Definieren von mehreren Referenz-Zustandsvektoren als Kombination von grenzwertrelevanten Ereignissen, denen jeweils eine spezifische Ziel-Grenzwerteinstellung zugeordnet ist. Diese Zuordnung zwischen grenzwertrelevanten Ereignissen und dem einzustellenden Ziel-Grenzwert oder Ziel-Grenzwertbereich wird in einer Zustandstabelle gespeichert.
- Erfassen von Messsignalen für alle oder ausgewählte Zustandsvariablen zur Berechnung eines Messsignalvektors
- Vergleich des berechneten Messsignalvektors mit den vordefinierten Referenz-Zustandsvektoren und bei Übereinstimmung:
- Steuerung und Überwachung des medizintechnischen Gerätes auf Einhaltung der Ziel-Grenzwerte. Mit anderen Worten soll der jeweilige charakteristische Ziel-Grenzwert, der in der Zustandstabelle für die erfassten Messsignale gespeichert ist, zur Geräteüberwachung verwendet werden. Der ursprüngliche Grenzwert kann somit zeitweise oder permanent mit einem neu eingestellten Ziel-Grenzwert oder Ziel-Grenzwertbereich überschrieben werden.

Damit erfolgt eine Überwachung des medizinischen Gerätes auf Einhaltung der systemzustandsabhängig eingestellten Ziel-Grenzwerte. Die Ziel-Grenzwerte sind dabei spezifisch und dynamisch an den jeweiligen Systemzustand angepasst.

Das erfindungsgemäße Verfahren ist ein Steuer- und Überwachungsverfahren. Es überwacht einen Systemzustand auf Einhaltung von Grenzwerten. Erfindungsgemäß werden die Grenzwerte dynamisch und ereignisgesteuert gesetzt. Damit entsteht der Vorteil, dass unnötige Fehlalarme vermieden, die Geräteüberwachung besser und schneller ausgeführt und Sondersituationen wesentlich besser bei der Überwachung berücksichtigt und abgebildet werden können. Darüber hinaus können auch wechselseitige Abhängigkeiten zwischen den grenzwertrelevanten Ereignissen auf einfache Weise berücksichtigt werden.

Grundsätzlich können unterschiedliche medizinische Geräte auf Einhaltung von Grenzwerten überwacht werden. Die bevorzugte Ausführungsform der Erfindung bezieht sich auf eine Drucküberwachung bei Blutbehandlungsgeräten, insbesondere Dialysegeräten. Demnach handelt es sich bei dem medizinischen Flüssigkeitskreislaufsystem bevorzugt um ein extrakorporales Blutkreislaufsystem, das von einem Dialysegerät gesteuert wird. Besonders bevorzugt ist die Überwachung von Dialysegeräten, die zur Hämodialyse, Hämofiltration, Hämodiafiltration oder Peritonealdialyse eingerichtet sind. Bevorzugt sind somit eine Drucküberwachung in dem medizinischen Flüssigkeitskreislaufsystem und insbesondere eine Drucküberwachung eines Hämodialysegerätes. Alternativ können jedoch ebenso andere medizintechnische Geräte überwacht werden, wie Plasmapheresegeräte, Geräte zur Unterstützung der Leberfunktion oder Herz-Lungenmaschinen.

Bei den Grenzwerten kann es sich um beliebige physikalische Grenzwerte des Flüssigkeitskreislaufsystems handeln. Vorzugsweise werden allerdings Druckwerte und Druckmesssignale auf Grenzwerteinhaltung überwacht.

Die Grenzwerte können erfindungsgemäß dynamisch an den Systemzustand angepasst und zeitweise oder permanent verändert werden. Sie werden in Abhängigkeit von vorbestimmten Ereignissen gesetzt, die ausgewertet werden. Dazu werden in einer Vorbestimmungsphase grenzwertrelevante Ereignisse definiert, wie z.B. Verabreichung eines Substituatsbolus im extrakorporalen Blutkreislauf oder andere Ereignisse, die zwar zu einer vorübergehenden Veränderung der Drucksituation im extrakorporalen System führen und die zwar außerhalb der ursprünglichen Grenzwertengrenzen liegen können, die aber in der speziellen Situation nicht relevant sind und keinen Alarm auslösen sollen. Um jedoch nicht auf eine Alarmierung bei Grenzwertüberschreitungen (oder Unterschreitungen) verzichten zu müssen, ist es wichtig, Systemzustände exakt auszuwerten, spezifische Bedingungen zu überprüfen und bei der Grenzwertsetzung zu berücksichtigen.

Während der Dialysebehandlung kann es unter Umständen notwendig werden, die an der Flüssigkeitsbilanzierung beteiligten sogenannte Bilanzpumpen, wie z.B. eine Dialysatpumpe, eine Substituatspumpe oder eine Ultrafiltrationspumpe, zeitweise zu stoppen. Dies ist beispielsweise dann der Fall, falls es bei den in den Pumpen eingelegten Schläuchen zu Fehlern (sogenanntes Kinking, Abknicken der Blutschläuche) oder Problemen kommt. Dies hat zur Folge, dass es nach dem zeitweiligen Abschalten einer der Bilanzpumpen beim Wiedereinschalten zu Druckimpulsen auf der Blutseite kommen, die nach dem Stand der Technik einen oder mehrere Alarme auslösen würden. Deshalb soll der Alarmgrenzwert erfindungsgemäß und abhängig von der aktuellen Situation (Ereignis: zeitweilige abgeschaltete Bilanzpumpe) zeitweise beispielsweise um 100mm HG angehoben werden. Die Druckpulse können insbesondere bei der Substituatspumpe durch den Flüssigkeitseintrag in den extrakorporalen Blutkreislauf entstehen. Eine andere Ursache kann bei zeitweisem Abschalten des Dialysatflusses in der Aufkonzentration von Hämatokrit im Dialysierfilter liegen, was einen erhöhten Flusswiderstand in Blutflussrichtung zur Folge hat und dementsprechend einen höheren arteriellen Blutdruck im extrakorporalen Blutkreislauf. Nach Wiedereinschalten des Dialysatflusses löst sich die Aufkonzentration verzögert.

In der Zustandstabelle werden alle oder ausgewählte grenzwertrelevante Ereignisse gespeichert und verwaltet, insbesondere in Form von Zustandsvariablen. Üblicherweise ist die Zustandstabelle vorbestimmt und im Laufe eines Überwachungsvorganges statisch. Alternativ kann die Zustandstabelle aber auch während eines Überwachungsvorganges verändert werden, etwa, falls neue grenzwertrelevante Ereignisse berücksichtigt und ausgewertet werden sollen oder falls eine abweichende Grenzwertzuordnung definiert werden soll. In der Zustandstabelle werden Kombinationen von grenzwertrelevanten Ereignissen und jeweils zugeordnetem Ziel-Grenzwert oder Ziel-Grenzwertbereich gespeichert. Dabei können zumindest zwei Ereignisse mit mathematischen Verknüpfungen (UND, ODER, UND NICHT etc.) kombiniert werden. Es ist auch möglich, für einen Gerätezustand (mit einer bestimmten Ereigniskombination) einen oder mehrere Ziel-Grenzwerte zu definieren. Des Weiteren können in der Tabelle Eingangs- und Ausgangsaktionen und bestimmte Bedingungen (z.B. Zeitdauer der veränderten Ziel-Grenzwertsetzung) definiert werden.

Eine Zustandsvariable repräsentiert ein grenzwertrelevantes Ereignis. Sie kann binär oder ein n-Tupel sein, wie z.B. "Blutfluss Erhöhung im extrakorporalen Blutkreislauf UND Flusswiderstand konstant". Dieses Ereignis wirkt sich insofern auf den Blutdruck im extrakorporalen Kreislauf aus, als dass sich dieser erhöht. Da diese Wirkung jedoch gewollt ist und nicht zu einem Alarm führen soll, muss der Ziel-Grenzwert entsprechend erhöht werden.

Ein Zustandsvektor ist die Gesamtheit aller Zustandsvariablen, die für den jeweiligen Grenzwert relevant sind. Dabei können auch einige Zustandsvariablen für mehrere Grenzwerte relevant sein.

Der Referenz-Zustandsvektor ist eine vorbestimmte Kombination von grenzwertrelevanten Ereignissen, für die eine spezifische Grenzwertänderung gegenüber dem ursprünglichen Grenzwert ausgeführt werden soll bzw. für die ein bestimmter Ziel-Grenzwert oder Ziel-Grenzwertbereich gesetzt werden soll. Damit wird sichergestellt, dass bei einem charakteristischen Ereignismuster aus vordefinierten grenzwertrelevanten Ereignissen (z.B. abgeschaltete Bilanzpumpe), eine bestimmte Ziel-Grenzwerteinstellung für ein bestimmtes Zeitintervall aktiviert werden soll (z.B. Erhöhen des Grenzwertes um einen vorbestimmten Faktor). Ferner können weitere Aktion definiert werden, die z.B. festlegen, wie lange die bestimmte Ziel-Grenzwerteinstellung aktiviert bleiben soll oder, ob diese nur nach einer weiteren Messsignalüberprüfung aktivierbar sein soll.

Gemäß einer bevorzugten Ausführungsform der Erfindung sind eine Vorbestimmungsphase und eine sich zeitlich anschließende Überwachungsphase vorgesehen. In der Vorbestimmungsphase werden die grenzwertrelevanten Ereignisse definiert und der zumindest eine Referenz-Zustandsvektor bestimmt. Der Referenz-Zustandsvektor definiert für eine charakteristische Kombination von Ereignissen oder Systemzuständen einen Ziel-Grenzwert, der spezifisch auf die jeweilige Ereigniskombination angepasst ist und üblicherweise von dem bislang gesetzten Grenzwert abweicht. In der Überwachungsphase werden für jedes Ereignis die jeweiligen Messwerte erfasst. Hier wird also überprüft, ob z.B. ein Substituatsbolus verabreicht worden ist (indem ein Substituatsbolus-Flag oder -Bit in einer Bitmaske gesetzt ist) oder ob eine Ultrafiltrationspumpe abgeschaltet werden musste oder wie das aktuelle Druckverhältnis an einem bestimmten Punkt im Blutkreislaufsystem ist. Diese Messwerte oder -signale werden über entsprechende Messsignalaufnehmer erfasst und gespeichert, um dann von einer Auswertelogik ausgelesen und mit den in der Vorbestimmungsphase bestimmten Referenz-Zustandsvektoren (üblicherweise sind dies mehrere) auf Übereinstimmung verglichen zu werden.

In einer Weiterbildung der Erfindung können sich die beiden Zeitphasen (Vorbestimmungsphase und Überwachungsphase) auch überschneiden. Somit ist es auch möglich, dass auch während der Überwachungsphase eine Vorbestimmung erfolgen kann, wenn nämlich geänderte (z.B. zusätzliche) grenzwertrelevante Ereignisse berücksichtigt werden sollen oder falls ein anderer oder zusätzlicher Referenz-Zustandsvektor definiert werden soll (z.B. ein anderer Ziel-Grenzwert für eine bestimmten Kombination von Ereignissen). Diese Ausführungsform eignet sich besonders als Trainingsphase, um die Ziel-Grenzwerte festzulegen.

Die Messsignale sind erfasste physikalische Werte des Flüssigkeitskreislaufsystems, insbesondere Druckmesswerte im arteriellen oder venösen Blutleitungen. Die Messsignale können sich jedoch auch auf andere physikalische Parameter beziehen, wie z.B. auf das geförderte Volumen, Temperatur etc. Die Messsignale oder Messwerte können durch ein analoges oder digitales Messverfahren generiert werden.

Gemäß einer bevorzugten Weiterbildung der Erfindung besteht eine Konfigurationsmöglichkeit dahingehend, wie lange und/oder unter welchen Bedingungen der geänderte Ziel-Grenzwert den ursprünglichen Grenzwert ersetzen soll. Hier kann beispielsweise eine bestimmte Zeitdauer eingestellt werden. Weiter kann überhaupt definiert werden, in welchen Zeitfenster oder Zeitintervall die ereignisgesteuerte Geräteüberwachung aktiviert oder deaktiviert sein soll. Dies hat den Vorteil, dass die Geräteüberwachung noch flexibler an den jeweiligen Anwendungsfall angepasst werden kann. Zudem können dadurch Notsituationen abgebildet werden, die eine möglichst alarmfreie Ausführung und Überprüfung erfordern.

Gemäß einem weiteren Aspekt der Erfindung ist eine Vielzahl von Referenz-Zustandsvektoren mit der jeweils zugeordneten Ziel-Grenzwerteinstellung vorgesehen. Die Vielzahl von Referenz-Zustandsvektoren wird in der Zustandstabelle gespeichert. Die Zustandstabelle umfasst zumindest zwei Zustandsvariablen, die grenzwertrelevante Ereignisse repräsentieren. Dies hat den Vorteil, dass eine Steuerung und Überwachung möglich wird, bei der einerseits die jeweiligen Ereignisse unabhängig voneinander auftreten und wobei andererseits sich die Ereignisse aber auch gegenseitig beeinflussen können, so dass wechselseitige Abhängigkeiten bei der Steuerung und Überwachung abgebildet werden. Dies ist zum Beispiel bei einem Aufweiten einer Druckgrenze der Fall (als Ziel-Grenzwert), obwohl diese Druckgrenze bereits durch ein anderes Ereignis aufgeweitet worden ist. Ebenso kann eine bestimmte Drucküberwachung deaktiviert und anschließend reaktiviert werden (als Zielzustand), obwohl die Drucküberwachung bereits durch ein anderes Ereignis deaktiviert ist und weiter deaktiviert bleiben soll. Interdependente grenzwertrelevante Ereignisse sind somit zur Einstellung eines Ziel-Grenzwertes in der Zustandstabelle codiert.

Gemäß einem weiteren Aspekt der Erfindung umfassen die Zustandsvariablen der Zustandstabelle binäre Zustandsvariablen und können somit als Bit einer Bitmaske repräsentiert werden oder als Flag, das gesetzt (Wert: 1) oder nicht gesetzt (Wert: 0) ist. Vorzugsweise ist jedes grenzwertrelevante Ereignis in jeweils einem Bit einer Bitmaske repräsentiert und wird so verarbeitet. Dabei wird vorzugsweise für die Einstellung jeweils eines Ziel-Grenzwertes eine eigene Bitmaske verarbeitet. Die Zustandsvariablen können des Weiteren einen physikalischen Parameter des medizinischen Flüssigkeitskreislaufsystems betreffen, wie z.B. eine Veränderung des Blutflusses bei einer Bilanzpumpe eines Dialysesystems oder ein von einer Blutpumpe verändert gefördertes Volumen.

Grundsätzlich kann die Drucküberwachung unterschiedliche ausgeführt werden. Im Wesentlichen gibt es zwei unterschiedliche Überwachungsarten: Erstens eine Überwachung auf einen von einer Bedienperson oder von dem Verfahren (automatisch) eingestellten Bereich (Druckfenster) und zweitens eine Überwachung auf einen festen oder von dem Verfahren eingestellten Bereich (Druckskala). Zudem können beide vorstehend genannte Alternativen auch kombiniert angewendet werden. Die Erfindung ist nicht auf eine Überwachungsart festgelegt. So kann die Einstellung der Grenzwerte eine Aufweitung oder eine Verkleinerung eines Grenzwertfensters sein sowie ein Nachführen eines unteren oder oberen Grenzwertes nach oben oder unten. Ebenso kann eine Neueinstellung umfasst sein.

Gemäß einem weiteren Aspekt der Erfindung werden die erfassten Messsignale immer im Verhältnis zum ursprünglichen Grenzwert gemessen und/oder gespeichert. Insbesondere wird bei einer Aufweitung des Druckfensters unabhängig von der ursprünglichen Aufweitungsanforderung der Wert der Aufweitung immer zum eigentlichen Druckgrenzwert gemessen.

Gemäß einem weiteren Aspekt der Erfindung können mehrere Gerätezustände von mehreren Flüssigkeitskreislaufsystemabschnitten (z.B. arterieller oder venöser Zweig) des Flüssigkeitskreislaufes parallel überwacht werden. Dabei kann jeweils ein Grenzwert jeweils einem Gerätezustand zugeordnet sein. Damit können vorteilhafterweise weitere Interdependenzen zwischen den zu überwachenden Ereignissen repräsentiert und bei der Überwachung berücksichtig werden.

Es kann konfiguriert werden, dass die Verfahrensschritte der Steuerung und der Überwachung (auch rekursiv) erneut und immer dann ausgeführt werden, sobald sich ein Messsignal zumindest eines grenzwertrelevanten Ereignisses geändert hat. Damit wird die Aktualität der Überwachung gewährleistet, so dass die Überwachung stets auf Basis des jeweils aktuellen Systemzustandes ausgeführt wird.

Gemäß einem weiteren Aspekt betrifft die Erfindung eine Grenzwertsetzvorrichtung zur ereignisgesteuerten Grenzwertsetzung auf Ziel-Grenzwerte in einem medizinischen Gerät, insbesondere einer Dialysemaschine, zur Steuerung eines Flüssigkeitskreislaufsystems. Die Grenzwertsetzvorrichtung umfasst:
- Eine Bestimmungseinrichtung, die zum Vorbestimmen von einer Vielzahl von grenzwertrelevanten Ereignissen bestimmt ist, die bei der Geräteüberwachung zu berücksichtigen sind und die in einer Zustandstabelle gespeichert werden und wobei die Bestimmungseinrichtung weiterhin zum Definieren von Referenz-Zustandsvektoren bestimmt ist, denen jeweils eine spezifische Ziel-Grenzwerteinstellung zugeordnet ist
- Einen Messsignalaufnehmer für jeweils einen Gerätezustand in dem medizinischen Flüssigkeitskreislaufsystem zum Erfassen von Messsignalen für alle oder ausgewählte der bestimmten grenzwertrelevanten Ereignisse zur Berechnung eines Messsignalvektors
- Eine Komparatorschaltung, die dazu bestimmt ist, den berechneten Messsignalvektor mit allen definierten Referenz-Zustandsvektoren zu vergleichen und bei Übereinstimmung eine Überwachungseinheit zu aktivieren
- Der Überwachungseinheit, die dazu bestimmt ist, die jeweilige dem Referenz-Zustandsvektor zugeordnete Ziel-Grenzwerteinstellung aus einem Speicher auszulesen und das medizinische Gerät auf Einhaltung der jeweils eingestellten Ziel-Grenzwerte zu überwachen
- Dem Speicher zur Speicherung der eingestellten Ziel-Grenzwerte.

Vorzugsweise umfasst die Grenzwertsetzvorrichtung auch den Speicher zur Speicherung der eingestellten Grenzwerte. Alternativ kann der Speicher als separater Baustein über eine Schnittstelle mit der Grenzwertsetzvorrichtung in Datenaustausch stehen.

Gemäß einem Aspekt der Erfindung ist die Grenzwertsetzvorrichtung in einen Geräteüberwachungsmonitor integriert oder an diesen als separate Vorrichtung angeschlossen ist, wobei der Geräteüberwachungsmonitor zur Geräteüberwachung auf Einhaltung von ereignisabhängigen Grenzwerten in einem medizinischen Flüssigkeitskreislaufsystem bestimmt ist.

Gemäß einem Aspekt der Erfindung handelt es sich bei dem Flüssigkeitskreislaufsystem um ein extrakorporales Blutbehandlungssystem. Es umfasst insbesondere eine Bilanzpumpe in Form einer Dialysatpumpe, einer Substituatspumpe und/oder einer Ultrafiltrationspumpe.

Wie vorstehend bereits erwähnt ist das Blutbehandlungssystem in einer bevorzugten Ausführungsform ein Dialysegerät, das insbesondere zur Hämodialyse, zur Hämofiltration, zur Hämodiafiltration und/oder zur Peritonealdialyse eingerichtet ist.

Üblicherweise ist es vorgesehen, dass jede Veränderung jeweils eines Grenzwertes gespeichert und somit nachvollziehbar wird. Dies hat den Vorteil, dass im Fehlerfall auch auf die ursprünglichen Grenzwerteinstellungen zurückgegriffen werden kann und diese wieder einspielbar sind.

Grundsätzlich können in dem Zeitraum, in dem die erfindungsgemäße Drucküberwachung aktiviert sein soll, verschiedene drucküberwachungsrelevante Ereignisse eintreten. Diese Ereignisse können unabhängig voneinander auftreten. Die Reaktion der Drucküberwachung ist in der Zustandstabelle codiert und umfasst z.B. folgende Aktionen:
- Deaktivieren des Druckfensters oder der Druckskala
- Setzen des Druckfensters auf die Druckskala
- Aufweiten des Druckfensters bezüglich des aktuell anliegenden Drucks
- Aufweiten des Druckfensters bezüglich des aktuell geltenden Druckgrenzwertes
- Verschieben des Druckfensters
- Neusetzen des Druckfensters.

Alle Ereignisse haben eine gemeinsame Zeitzählung zur (zeitlich begrenzten) Beeinflussung der Drucküberwachung. Damit kann eine Synchronisierung der unabhängig auftretenden Ereignisse erreicht werden. Alle Ereignisse nutzten vorzugsweise eine gemeinsame Variable, in der der Wert der Aufweitung gespeichert wird. Sämtliche Flags, Zeiten und Aufweitungswerte oder Zielgrenzwerte können jederzeit durch ein (neues) Ereignis zurückgesetzt werden. In der bevorzugten Ausführungsform ist es vorgesehen, dass die ursprünglichen Grenzwerte mit den Ziel-Grenzwerten für eine bestimmte Dauer und/oder unter bestimmten Bedingungen überschrieben werden. Der Überschreibungsvorgang kann - aus Sicherheitsgründen - von dem Erfassen eines Bestätigungssignals eines Anwenders abhängig gemacht werden.

Weiterhin betrifft die Erfindung ein medizintechnisches Gerät mit einem Steuergerät. Dabei kann das medizintechnische Gerät ein Dialysegerät, insbesondere zur Hämodialyse, zur Hämofiltration, zur Hämodiafiltration und/oder zur Peritonealdialyse sein. Das Steuergerät umfasst die Komparatorschaltung und die Überwachungseinheit sowie eine Eingangsschnittstelle zum Einlesen von Messsignalen. Die Messsignale können von Messsignalaufnehmern erfasst werden. Des Weiteren dient die Eingangsschnittstelle zum Einlesen von zumindest einem Zustandsvektor und von zumindest einem Referenz-Zustandsvektor. Vorzugsweise umfasst das Steuergerät noch den Speicher, in dem Ziel-Grenzwerte, Zustandsvektoren und/oder Referenz-Zustandsvektoren und Übereinstimmungen und/oder Abweichungen von Ziel-Grenzwert und jeweils ursprünglichem Grenzwert gespeichert und auslesbar sein können. Das Steuergerät kann zumindest teilweise als internes Modul in das Dialysegerät integriert oder als externes Modul permanent oder zeitphasenweise dem Dialysegerät zugeschaltet werden. Vorzugsweise kann das Steuergerät noch eine oder mehrere Benutzerschnittstellen zur Ein- und Ausgabe von Daten umfassen.

Die vorstehend beschriebenen, erfindungsgemäßen Ausführungsformen des Verfahrens können auch als Computerprogrammprodukt mit einem Mikroprozessor-Computerprogramm ausgebildet sein, wobei der Computer zur Durchführung des oben beschriebenen, erfindungsgemäßen Verfahrens veranlasst wird, wenn das Computerprogramm auf dem Computer bzw. auf einem Prozessor des Computers ausgeführt wird. Eine alternative Aufgabenlösung besteht auch in einem Computerprogramm mit Computer-Programmcode zur Durchführung aller Verfahrensschritte des beanspruchten oder oben beschriebenen Verfahrens, wenn das Computerprogramm auf dem Computer ausgeführt wird. Dabei kann das Computerprogramm auch auf einem maschinenlesbaren Speichermedium gespeichert sein. Eine alternative Aufgabenlösung sieht ein Speichermedium vor, das zur Speicherung des vorstehend beschriebenen, computer-implementierten Verfahrens bestimmt ist und von einem Computer oder Prozessor lesbar ist.

Es liegt im Rahmen der Erfindung, dass nicht alle Schritte des Verfahrens zwangsläufig auf ein und demselben Bauteil oder derselben Computerinstanz ausgeführt werden müssen, sondern sie können auch auf unterschiedlichen Computerinstanzen ausgeführt werden. Auch kann die Abfolge der Verfahrensschritte gegebenenfalls variiert werden.

Darüber hinaus ist es möglich, dass einzelne Abschnitte des vorstehend beschriebenen Verfahrens in einer verkaufsfähigen Einheit und die restlichen Komponenten in einer anderen verkaufsfähigen Einheit - sozusagen als verteiltes System - ausgeführt werden können. So ist es insbesondere möglich, die Verfahrensschritte des Vorbestimmens und Definierens in einem ersten Modul und das Erfassen der Messsignale in einem zweiten Modul und das Vergleichen in einem dritten Modul auszuführen. Das dritte Modul kann insbesondere als Steuergerät zur Steuerung eines Dialysegerätes oder eines anderen medizintechnischen Gerätes in das Gerät selbst integriert sein oder diesem durch entsprechende Datenverbindung zugeschaltet werden.

In der folgenden detaillierten Figurenbeschreibung werden nicht einschränkend zu verstehende Ausführungsbeispiele mit deren Merkmalen und weiteren Vorteilen anhand der Zeichnung besprochen.

### Kurze Beschreibung der Figuren

Fig. 1 zeigt eine schematische Ansicht eines bekannten Hämodialysegerätes, das mit dem erfindungsgemäßen Verfahren betrieben und überwacht wird,
Fig. 2 zeigt in schematischer Form eine Zustandstabelle mit darin gespeicherten Datensätzen und Messwerten,
Fig. 3 zeigt ein Ablaufdiagramm gemäß einer bevorzugten Ausführungsform des erfindungsgemäßen Verfahrens,
Fig. 4 ist eine schematische Übersicht einer Grenzwertsetzvorrichtung gemäß einer bevorzugten Ausführungsform der Erfindung und
Fig. 5 zeigt eine schematische Ansicht eines Dialysegerätes mit einem Steuergerät gemäß einer bevorzugten Ausführungsform der Erfindung.

### Detaillierte Beschreibung der Erfindung

Blutbehandlungsgeräte sind medizintechnische Geräte, bei denen das Blut eines Patienten in der Regel extrakorporal behandelt wird. Beispielsweise können dem Blut Medikamente zugesetzt oder ihm Blutbestandteile entzogen werden, es kann erwärmt oder gekühlt werden. Zu diesem Zweck wird das Blut oftmals außerhalb des Körpers mit einer Blutpumpe gepumpt, behandelt und dem Körper des Patienten wieder zurückgegeben. Ein solcher Blutkreislauf heißt extrakorporaler Blutkreislauf.

Im Folgenden soll die Erfindung anhand des Beispiels eines Dialysegerätes als Ausführungsform eines medizintechnischen Geräts erklärt werden.

Weitere Blutbehandlungsgeräte sind beispielsweise Geräte zur Unterstützung der Herz-Lungentätigkeit, wie Blutoxygenatoren, oder Geräte zur Unterstützung der Leberfunktion, die Bluttoxine durch Adsorption aus dem Blut entfernen. Oftmals kombinieren Geräte zur Unterstützung der Leberfunktion Dialyseverfahren und Adsorptionsverfahren in einem Gerät, wie beispielsweise das Gerät Prometheus der Anmelderin.

Bei Dialysegeräten wird ein Fluid eines Patienten über eine Fluidleitung einer Fluidbehandlungskomponente zugeführt, durch die Fluidbehandlungskomponente behandelt und über die Fluidleitung, die in einen arteriellen und einen venösen Zweig aufgeteilt werden kann, wieder an den Patienten zurückgegeben. Beispiele für solche Blutbehandlungsgeräte sind insbesondere Hämodialysegeräte. Ein solches Blutbehandlungsgerät ist Gegenstand der DE 198 49 787 C1 der Anmelderin, deren Inhalt hiermit vollumfänglich im Offenbarungsgehalt der vorliegenden Anmeldung einbezogen wird.

Die Dialyse ist ein Verfahren zur Blutreinigung von Patienten mit akuter oder chronischer Niereninsuffizienz. Grundsätzlich unterscheidet man hierbei zwischen Verfahren mit einem extrakorporalen Blutkreislauf, wie--der Hämodialyse, der Hämofiltration oder der Hämodiafiltration und der Peritonealdialyse, die keinen extrakorporalen Blutkreislauf aufweist.

Das Blut wird bei der Hämodialyse einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration der Blutelektrolyte in der Dialysierflüssigkeit entspricht dabei der Konzentration im Blut eines Gesunden. Während der Behandlung werden das Blut des Patienten und die Dialysierflüssigkeit an beiden Seiten der semipermeablen Membran im Allgemeinen im Gegenstrom mit einer vorgegebenen Flussrate vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Wird an der Dialysemembran ein Druckgradient von der Blutseite zur Dialysatseite aufgebaut, beispielsweise durch eine Pumpe, die flussabwärts des Dialysefilters auf der Dialysatseite Dialysat aus dem Dialysatkreislauf entzieht, tritt Wasser aus dem Patientenblut über die Dialysemembran in den Dialysatkreislauf über. Dieser Vorgang der Ultrafiltration führt zu einer gewünschten Entwässerung des Patientenbluts.

Bei der Hämofiltration wird dem Patientenblut durch Anlegen eines Transmembrandruckes im Dialysator Ultrafiltrat entzogen, ohne dass Dialysierflüssigkeit auf der dem Patientenblut gegenüber liegenden Seite der Membran des Dialysators vorbeigeführt wird. Zusätzlich kann dem Patientenblut eine sterile und pyrogenfreie Substituatslösung zugesetzt werden. Je nachdem, ob diese Substituatslösung stromaufwärts des Dialysators zugesetzt wird oder stromabwärts, spricht man von Prä-oder Postdilution. Der Stoffaustausch erfolgt bei der Hämofiltration konvektiv.

Die Hämodiafiltration kombiniert die Verfahren der Hämodialyse und der Hämofiltration. Es findet sowohl ein diffusiver Stoffaustausch zwischen Patientenblut und Dialysierflüssigkeit über die semipermeable Membran eines Dialysators statt, als auch eine Abfiltrierung von Plasmawasser durch einen Druckgradienten an der Membran des Dialysators.

Die Verfahren der Hämodialyse, der Hämofiltration und der Hämodiafiltration werden in der Regel mit automatischen Hämodialysegeräten durchgeführt, wie sie beispielsweise von der Anmelderin unter der Bezeichnung 5008 vertrieben werden.

Die Plasmapherese, ist ein Blutbehandlungsverfahren, bei dem das Patientenblut in das Blutplasma und seine korpuskularen Bestandteile (Zellen) aufgetrennt wird. Das abgetrennte Blutplasma wird gereinigt oder durch eine Substitutionslösung ersetzt und das gereinigte Blutplasma oder die Substitutionslösung dem Patienten zurückgegeben.

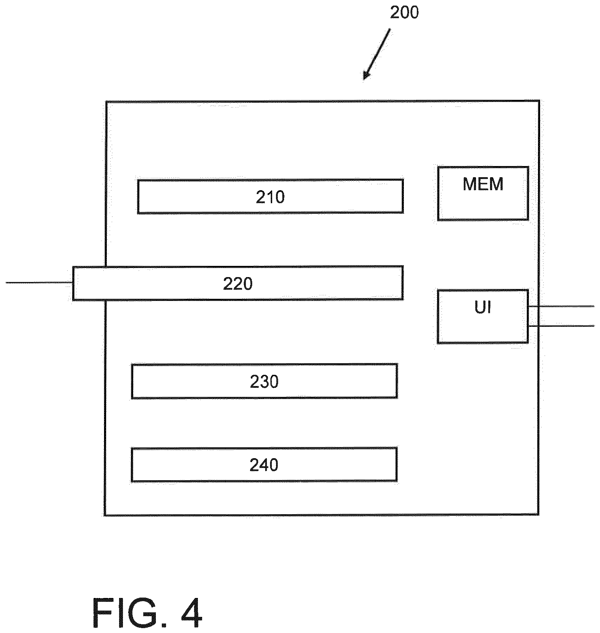

Dialysegeräte, als Beispiel für komplexe medizintechnische Geräte, haben umfangreiche Funktionen. Um diese Funktionen zu steuern, sind medizintechnische Geräte wie Dialysegeräte mit zumindest einer Steuervorrichtung ausgerüstet. Diese kann als CPU (central processing unit) oder Mikrokontroller ausgeführt sein, die von Softwareprogrammen programmiert wird. Die Bedienung solcher Geräte geschieht häufig über Touchscreen Displays UI (wie schematisch in Fig. 4 dargestellt). Ein solches Touchscreen Display UI kombiniert in einer gemeinsamen Oberfläche eine Ein-und eine Ausgabevorrichtung, in dem es eine berührungsempfindliche Oberfläche bereitstellt, mit der Bedienereingaben detektierbar sind.

Dialysepflichtige Patienten müssen sich regelmäßig mehrstündigen Dialysebehandlungen unterziehen. Hierbei wird die Dauer der Dialyse individuell von einem Arzt festgesetzt, wobei in die Berechnung der Dauer die Parameter der Dialysebehandlung und die physiologischen Parameter des Patienten eingehen.

So wird beispielsweise berücksichtigt, mit welcher Überwässerung der Patient zu Beginn der Behandlung erscheint. Die Überwässerung ist ein Maß für das überschüssige Wasservolumen bzw. Gewicht im Blut und Gewebe des Patienten. Patienten, welche an Niereninsuffizienz leiden, können das mit der Nahrung aufgenommene Wasser nicht oder nur unzureichend ausscheiden. Dieses Wasser lagert sich im Blut und im Gewebe des Patienten an und muss durch die Dialysebehandlung über Ultrafiltrationsverfahren dem Patienten entzogen werden. Die Überwässerung wird in der Regel durch Wiegen des Patienten und Vergleich mit einem Referenzgewicht, dem sogenannten Trockengewicht, bestimmt. Die Ultrafiltration des Blutes bei einer Dialysebehandlung kann mit einer konstanten Ultrafiltrationsrate, intermittierend oder entlang eines Ultrafiltrationsprofils erfolgen. Ultrafiltrationsrate, Ultrafiltrationsvolumen und Ultrafiltrationsprofil sind Parameter, die die Dauer der Dialysebehandlung beeinflussen.

Die Dialysebehandlung mit Dialysegeräte unterliegt umfangreichen Sicherheitsvorkehrungen, um die Patientengesundheit nicht zu gefährden. Deswegen sind Dialysegeräte mit einer Vielzahl von Sensoren ausgestattet, die unterschiedliche Parameter der überwachen. Im Falle von Grenzwertverletzungen kann die Steuerung des Dialysegeräts in die Behandlung eingreifen. Zu den überwachten Parametern gehört insbesondere der Blutdruck im arteriellen und venösen Zweig des extrakorporalen Blutkreislaufes.

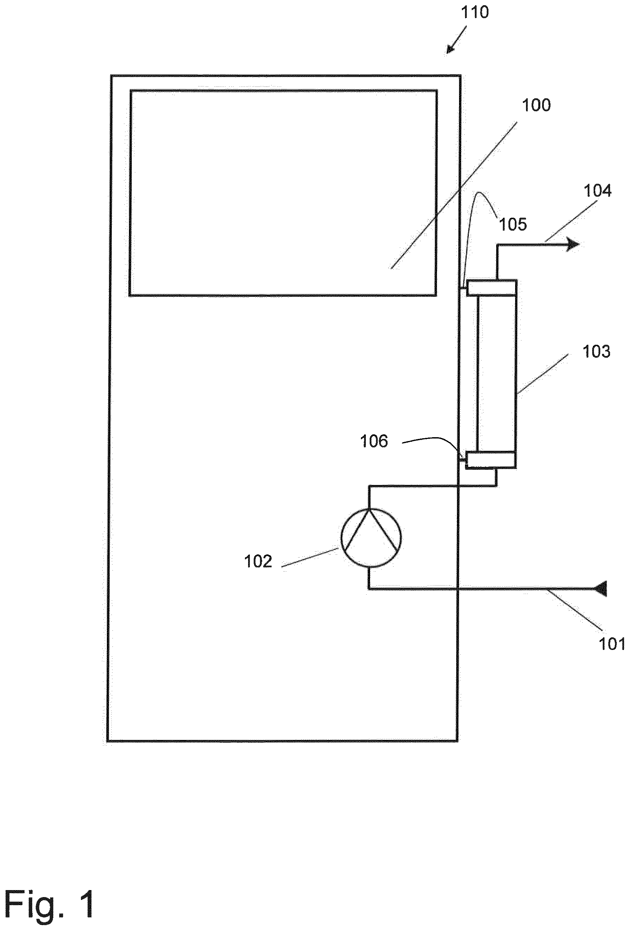

In **Figur 1** ist schematisch ein als Hämodialysegerät 110 ausgeführtes Blutbehandlungsgeräts, als Beispiel für ein medizintechnisches Gerät wie es im Stand der Technik bekannt ist, dargestellt.

Das Hämodialysegerät 110 umfasst Teile eines extrakorporalen Blutkreislaufs mit einer arteriellen Blutleitung 101, die Blut eines Patienten (nicht dargestellt) ableitet.

Die Blutpumpe 102 fördert das Blut über den in sie eingelegten Schlauchabschnitt durch einen Dialysator 103, der mit einer semipermeablen Membran ausgestattet ist, die den extrakorporalen Blutkreislauf von einem Dialysatkreislauf semipermeabel trennt. Über die venöse Leitung 104 wird das behandelte Blut dem Patienten zurückgegeben. Die verwendeten Leitungen und Schlauchabschnitte, insbesondere auch die Teile, die den extrakorporalen Blutkreislauf ausbilden, sind in der Regel Einmalteile, die nach Gebrauch verworfen werden. Über die Dialysatleitungen 105 und 106 wird Dialysat durch den Dialysefilter 103 gepumpt, wo es über die semipermeable Membran des Dialysefilters 103 zu einem diffusiven Stoffaustausch mit dem Blut des Patienten kommt. Wird zusätzlich ein Druckgradient von der Blutseite des Dialysefilters zur Dialysatseite des Patienten aufgebaut, wird Plasmawasser aus dem Blut in das Dialysat abgepresst. Das Blut des Patienten kann so entwässert werden. Das Dialysat wird in dem Hämodialysegerät 110 hergestellt und nach Gebrauch verworfen.

Das Hämodialysegerät 110 ist mit einem in Figur 1 nicht dargestellten Steuergerät ausgestattet, welches so programmiert werden kann, dass es im Zusammenwirken mit Vorrichtungen des Hämodialysegerätes 110 die Verfahren nach der Lehre der Erfindung ausführen kann. Das Steuergerät kann eine Grenzwertsetzvorrichtung 200 umfassen.

Das Steuergerät dient zur Steuerung und/oder Überwachung des Hämodialysegerätes 110 und/oder des extrakorporalen Flüssigkeitskreislaufsystems. Insbesondere werden mittels der Grenzwertsetzvorrichtung 200 vordefinierte Grenzwerte auf Einhaltung überwacht.

**Figur 4** zeigt auf schematische Weise eine Grenzwertsetzvorrichtung 200. Sie umfasst mehrere elektronische Bauteile, die alle über entsprechenden Datenleitungen (nicht dargestellt) in Verbindung stehen. Eine Bestimmungsvorrichtung 210 dient zum Vorbestimmen von einer Vielzahl von grenzwertrelevanten Ereignissen. Die Ereignisse definieren einen spezifischen Systemzustand, also mit anderen Worten eine spezifische Kombination von Zuständen (vereinfacht z.B.: "Stopp der Bilanzpumpen" UND "Verabreichung eines Substituatsbolus"). Für alle möglichen Systemzustände bzw. Ereigniskombinationen wird jeweils ein Zustandsvektor ZV in einer Zustandstabelle T definiert. Alle Ereignisse und Ereigniskombination werden jeweils als ein Zustandsvektor ZV gespeichert und bei der Geräteüberwachung berücksichtigt. Die Bestimmungseinrichtung 210 ist weiterhin zum Definieren von Referenz-Zustandsvektoren R-ZV bestimmt, denen jeweils eine spezifische Ziel-Grenzwerteinstellung Z-GW zugeordnet ist.

Die Grenzwertsetzvorrichtung 200 umfasst weiter einen Messsignalaufnehmer 220 für jeweils einen Gerätezustand (Kombination von Ereignissen) in dem medizinischen Flüssigkeitskreislaufsystem zum Erfassen von Messsignalen für alle oder ausgewählte der bestimmten grenzwertrelevanten Ereignisse zur Berechnung eines Messsignalvektors.

Die Grenzwertsetzvorrichtung 200 umfasst weiter eine Komparatorschaltung 230, die dazu bestimmt ist, den berechneten Messsignalvektor mit den jeweiligen, insbesondere mit allen, definierten Referenz-Zustandsvektoren R-ZV zu vergleichen und bei Übereinstimmung eine Überwachungseinheit 240 zu aktivieren.

Die Grenzwertsetzvorrichtung 200 umfasst weiter eine Überwachungseinheit 240, die dazu bestimmt ist, die jeweilige dem Referenz-Zustandsvektor R-ZV zugeordnete Ziel-Grenzwerteinstellung Z-GW aus einem Speicher MEM auszulesen und das medizinische Gerät 110 auf Einhaltung der jeweils eingestellten Ziel-Grenzwerte Z-GW zu überwachen.

Der Speicher MEM ist vorzugweise direkt in die Grenzwertsetzvorrichtung 200 integriert und dient zur Speicherung der eingestellten Ziel-Grenzwerte Z-GW und Grenzwerte.

Wie in Figur 4 dargestellt, weist die Grenzwertsetzvorrichtung 200 noch Ein-und Ausgabeschnittstellen auf. Über eine Eingangsschnittstelle werden Messsignale oder Messwerte, die an Sensoren erfasst werden an den Messsignalaufnehmer 220 weitergeleitet oder unmittelbar von ihm erfasst. Eine Benutzerschnittstelle, insbesondere ein Touchscreen Display, UI dient zur Ein- und Ausgabe von für die Grenzwertsetzung relevanten Daten und Bestätigungssignalen seitens des Anwenders.

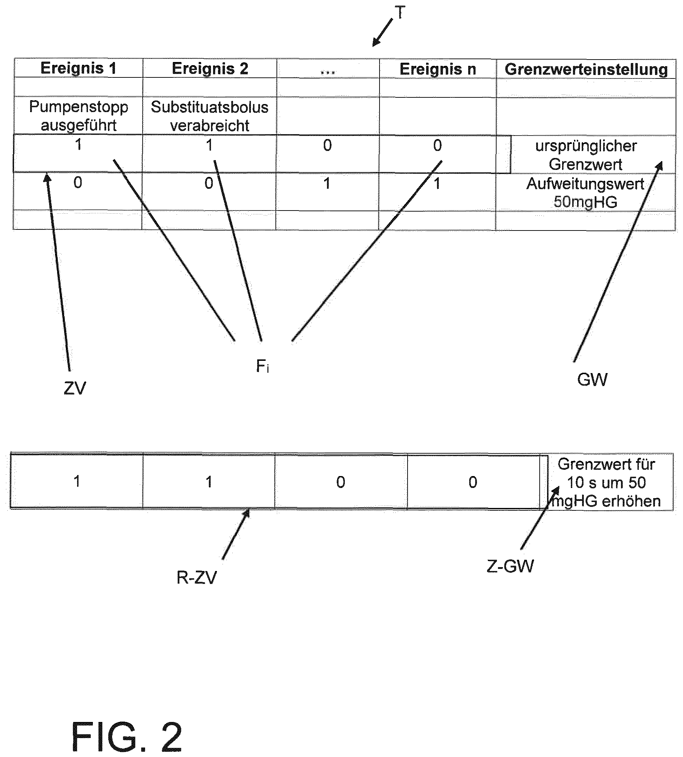

Im Zusammenhang mit **Figur 2** soll im Folgenden nochmals die Zustandstabelle T mit den darin gespeicherten Datensätzen erläutert werden. Als grenzwertrelevantes Ereignis werden vorzugweise singuläre Ereignisse erfasst, die zwei Systemzustände einnehmen können und somit binär repräsentiert werden können. Komplexere Ereignisse werden in eine Kombination von singulären Ereignissen zergliedert. Vorzugweise können diese Ereignisse deshalb als einzelne Flags (in Figur 2 als Fᵢ gekennzeichnet) oder Bits einer Bitmaske gespeichert werden. Der Begriff "Kombination" umfasst alle mengentheoretischen Abbildungen, wie UND, ODER, NICHT. Als exemplarisches Beispiel ist in Figur 2 für das Ereignis 1 "Pumpenstopp ausgeführt?" und für das Ereignis 2 "Substituatsbolus verabreicht?" angegeben. Selbstverständlich gibt es hier eine Reihe von weiteren Ereignissen, die stellvertretend in Figur 2 mit "Ereignis n" gekennzeichnet sind. Eine Kombination von Ereignissen wird als Zustandsvektor ZV gespeichert. Einem Zustandsvektor ZV kann ein bestimmter (ursprünglicher) Grenzwert zugeordnet sein. Eine charakteristische Kombination von Ereignissen wird als Referenz-Zustandsvektor R-ZV bezeichnet. Dem Referenz-Zustandsvektor R-ZV ist jeweils eine bestimmte Ziel-Grenzwerteinstellung Z-GW zugeordnet. Sobald die Komparatorschaltung 230 erfasst, dass ein Messsignalvektor (also eine Kombination von erfassten Messwerten oder Messsignalen) mit einem der Referenz-Zustandsvektoren R-ZV übereinstimmt, kann die vordefinierte jeweilige Ziel-Grenzwerteinstellung Z-GW verwendet werden. In diesem Fall kann der ursprünglich vorgesehene Grenzwert GW mit dem Ziel-Grenzwert Z-GW für ein bestimmtes Zeitintervall oder permanent überschrieben werden.

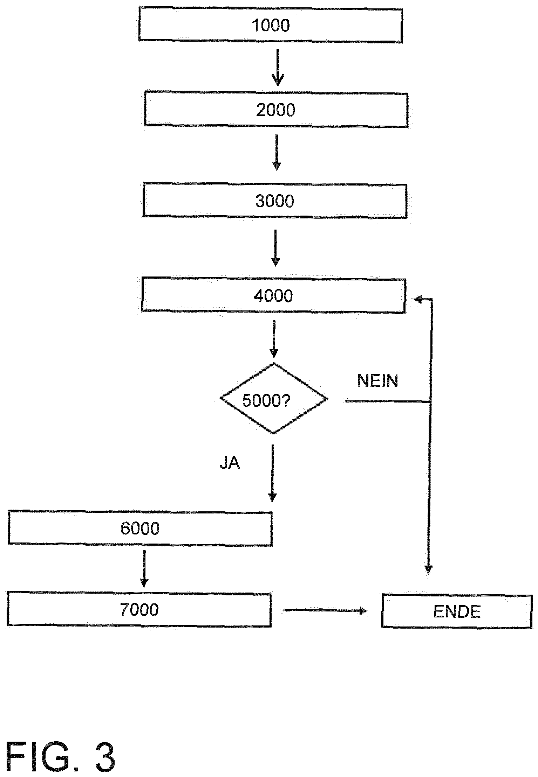

**Figur 3** zeigt den Ablauf eines Verfahrens gemäß einem Ausführungsbeispiel. Nach dem Start des Verfahrens werden in Schritt 1000 alle grenzwertrelevanten Ereignisse vorbestimmt. In Schritt 2000 werden die charakteristischen Referenz-Zustandsvektoren R-ZV definiert, für die zugeordnete Ziel-Grenzwerteinstellungen Z-GW verwendet werden sollen. Die Schritte 1000 und 2000 können in einer Vorbereitungsphase ausgeführt werden und sind der eigentlichen Grenzwertsetzung und Überwachung vorgeschaltet. Die Überwachungsphase kennzeichnet sich durch die Ausführung der Schritte 3000 bis 7000. Schritt 3000 dient zum Erfassen von Messwerten und Messsignalen für die grenzwertrelevanten Ereignisse. Vorzugsweise handelt es sich dabei um gemessene Druckverhältnisse. Es können aber auch andere physikalische Parameter erfasst werden. Aus der Kombination von erfassten Messwerten wird ein Messsignalvektor generiert, der in Schritt 4000 mit den definierten Referenz-Zustandsvektoren R-ZV auf Übereinstimmung verglichen wird. Sobald in Schritte 5000 eine Übereinstimmung erfasst wird, wird die jeweils zugeordnete Ziel-Grenzwerteinstellung Z-GW aus der Tabelle T ausgelesen und zur Steuerung 6000 des Hämodialysegerätes 110 verwendet. In Schritt 7000 wird die Überwachung des Gerätes 110 unter Verwendung der Ziel-Grenzwerte Z-GW ausgeführt. Falls in der Abfrage 5000 keine Übereinstimmung erkannt werden kann, kann das Verfahren enden oder es verzweigt und wiederholt die die Schritte 4000 und folgende.

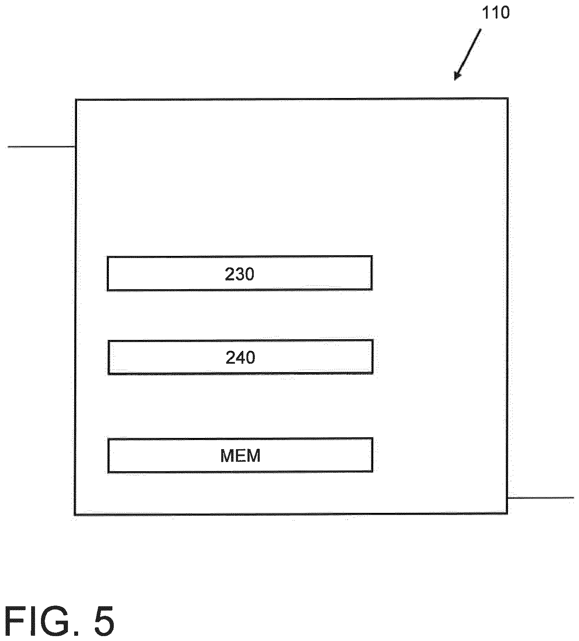

**Figur 5** zeigt schematisch ein Dialysegerät 110, das mit einem Steuergerät 250 ausgestattet ist. Dabei kann das Dialysegerät 110 insbesondere zur Hämodialyse, zur Hämofiltration, zur Hämodiafiltration und/oder zur Peritonealdialyse geeignet sein. Das Steuergerät 250 umfasst die Komparatorschaltung 230 und die Überwachungseinheit 240 sowie eine Eingangsschnittstelle zum Einlesen von Messsignalen. Die Messsignale können von Messsignalaufnehmern erfasst werden. Des Weiteren dient die Eingangsschnittstelle (nicht dargestellt) zum Einlesen von zumindest einem Zustandsvektor und von zumindest einem Referenz-Zustandsvektor. Vorzugsweise umfasst das Steuergerät 250 noch den Speicher MEM, in dem Ziel-Grenzwerte Z-GW, Zustandsvektoren und/oder Referenz-Zustandsvektoren R-ZV und Übereinstimmungen und/oder Abweichungen von Ziel-Grenzwert Z-GW und jeweils ursprünglichem Grenzwert gespeichert und auslesbar sind. Das Steuergerät 250 kann zumindest teilweise als internes Modul in das Dialysegerät 110 integriert oder als externes Modul permanent oder zeitphasenweise dem Dialysegerät 110 zugeschaltet werden. Vorzugsweise kann das Steuergerät 250 noch eine oder mehrere Benutzerschnittstellen UI zur Ein- und Ausgabe von Daten umfassen.

Im Folgenden wird die erfindungsgemäße Grenzwertsetzung anhand von ausgewählten Anwendungsszenarien beispielhaft erläutert.

So kann es während der Dialyse beispielsweise erforderlich sein, einen Subsituatsbolus zu verabreichen. Ein Substituatsbolus ist eine zeitweise Blutverdünnung durch die Gabe eines Bolus einer Substitutionsflüssigkeit (Substituat), die stromaufwärts oder stromabwärts des Dialysefilters erfolgen kann. Diese zusätzliche Blutverdünnung führt in Folge der Veränderung der Viskosität zu einer Veränderung der Druckverhältnisse im venösen und arteriellen Zweig des extrakorporalen Kreislaufs. Eine solche Maßnahme wird insbesondere dann durchgeführt, wenn der Blutdruck des Patienten zu entgleiten droht. Die Zugabe des Substituats erfolgt unmittelbar in den extrakorporalen Blutkreislauf, was eine kurzzeitige Druckerhöhung in diesem zur Folge hat, die sich nach der Vermischung des Bolus mit dem Patientenblut im vaskulären System stabilisiert. Um unnötige Alarme in Folge des Druckimpulses zu vermeiden, kann der Grenzwert, der im extrakorporalen Blutkreislauf gemessen wird, mittels der erfindungsgemäßen Grenzwertsetzvorrichtung 200 kurzzeitig (beispielsweise für die nächsten 100ml Blutfördervolumen) erhöht werden.

Erfindungsgemäß wird bei einem Substituatsbolus - entgegen der ursprünglichen (Vor-) Einstellung der Grenzwerte (zur Steuerung eines Alarmsignals) - beispielsweise die obere Grenze des Drucküberwachungsfensters während des Bolus' und nach Abschluss des Bolus' von der Grenzwertsetzvorrichtung für weitere 100ml gefördertes Blutvolumen um 50 mmHg erhöht.

Ein anderes Beispiel betrifft die Grenzwertsetzvorrichtung und die erfindungsgemäße Steuerung bei einem Pumpenstopp. Während der Dialysebehandlung kann es notwendig werden, die an der Flüssigkeitsbilanzierung beteiligten Pumpen wie Dialysatpumpe, Substituatspumpe oder Ultrafiltrationspumpe zeitweise zu stoppen. Diese Pumpen sind in dieser Patentanmeldung unter dem Begriff Bilanzpumpen zusammengefasst. Die Bilanzierung bei Hämodialysebehandlungen sorgt im Wesentlichen dafür, dass der Volumenstrom an Dialysierflüssigkeit (Dialysat), der in den Dialysator eintritt, dem Volumenstrom entspricht, der aus dem Dialysator austritt. So wird sichergestellt, dass ohne Ultrafiltrationspumpe, die zusätzlich Dialysierflüssigkeit stromabwärts des Dialysefilters aus dem Dialysierflüssigkeitskreislauf entfernt, kein Plasmawasser aus dem Blut des Patienten über die Dialysierfiltermembran auf die Dialysatseite abfiltriert wird. Ein wesentliches Ziel der Dialyse ist aber auch der Entzug von überschüssigem Wasser aus dem Patientenblut, der infolge seiner Niereninsuffizienz mit der Nahrung aufgenommenes Wasser nur unzureichend ausscheiden kann. Dieser Vorgang der Ultrafiltration muss aber genau erfolgen, da der Flüssigkeitshaushalt des Patienten gesundheitsrelevant ist, insbesondere kritisch für dessen Kreislaufstabilität. Durch die langen Dialysierzeiten (i.d.R. ca. 4 Stunden pro Sitzung) könnten sich größere Fehler im Ultrafiltrationsvorgang akkumulieren. Deswegen werden für die Ultrafiltrationspumpen hochgenaue Pumpen eingesetzt. Durch den Bilanziervorgang wird dafür Sorge getragen, dass tatsächlich nur durch die Ultrafiltrationspumpe ein Ultrafiltrationsvorgang in Gang gebracht werden kann, und die dem Patientenblut entzogene Wassermenge dem Volumen entspricht, welches die Ultrafiltrationspumpe fördert. Ebenfalls in die Bilanzierung mit einzubeziehen ist das in den Blutkreislauf eingetragene Volumen durch die Substituatspumpe. An der Flüssigkeitsbilanz sind somit die Dialysatpumpe, die Ulrafiltrationspumpe und die Substituatspumpe beteiligt.

Beim Stopp der Bilanzpumpen werden grenzwertrelevante Ereignisse erfasst und zur Grenzwertsetzung ausgewertet. So wird z.B. die obere Grenze des Drucküberwachungsfensters während des Stopps und nach Wiederanfahren von der Grenzwertsetzvorrichtung 200 für weitere 80ml gefördertes Blutvolumen um 100 mmHg erhöht.

Ein weiteres Beispiel betrifft eine Kombination von grenzwertrelevanten Ereignissen, wie die Verabreichung eines Substituatsbolus und einen nachfolgenden Bilanzpumpenstopp: Nachdem nach einem Substituatsbolus 40ml Blut gefördert wurden, werden die Bilanzpumpen durch den Anwender gestoppt. Da der Aufweitungswert jetzt z.B. um 50mmHg größer ist als beim Substituatsbolus, wird der Grenzwert mit der erfindungsgemäßen Steuerung um diese 50mmHg erhöht. Dieser Wert bleibt dann für die nächsten 80ml des geförderten Volumens bestehen, und der Grenzwert wird nach Erreichen dieses Volumens von der Grenzwertsetzvorrichtung 200 auf den ursprünglichen Grenzwert zurückgesetzt.

Die erfindungsgemäße Grenzwertsetzvorrichtung 200 steuert die Grenzwertsetzung auch auf Basis einer Kombination von charakteristischen Ereignissen oder Zuständen. Beispielsweise hat die Erhöhung des Blutflusses unmittelbar Auswirkung auf den extrakorporalen Blutdruck (er wird ebenfalls erhöht). Diese Wirkung ist allerdings beabsichtigt, so dass die obere Grenze des Drucküberwachungsfensters von der Grenzwertsetzvorrichtung 200 bei einer Erhöhung des Blutflusses für 10 s deaktiviert und anschließend so um den dann herrschenden Druck gesetzt, wie es vor der Erhöhung des Blutflusses um den zum damaligen Zeitpunkt herrschenden Druck gesetzt war.

Druckschwankungen, die zu einer geänderten Grenzwertsetzung führen, können auch aufgrund von Druckeinträgen in der venösen Tropfkammer entstehen. Das Blut des Patienten gelangt nach dem Dialysator i.d.R. in eine venöse (sogenannte Tropf-) Kammer, in der dem Blut beispielsweise Substituat oder Medikamente zugemischt, es entgast werden kann und in der Partikelfilter angeordnet sind, um unerwünschte Partikel zurückzuhalten. Der Blutpegel der venösen Tropfkammer wird überwacht und geregelt. Die Einstellung des Blutpegels kann beispielsweise pneumatisch erfolgen, in dem Druckluft aus der Maschine in die Tropfkammer geleitet wird. Ein derartiger Druckeintrag in den extrakorporalen Blutkreislauf kann zu kurzzeitigen Druckschwankungen führen. Um unerwünschte Alarme zu vermeiden, kann die Drücküberwachung für die Dauer der Regelung (beispielsweise 10 s) ausgesetzt werden. Dieser Zustand wird erfindungsgemäß berücksichtigt, indem das Drucküberwachungsfenster von der Grenzwertsetzvorrichtung 200 während des Pegelsenkens in der venösen Kammer und während der anschließenden 10s für 10 s deaktiviert wird.

Ein weiteres Beispiel des erfindungsgemäßen Grenzwertsetzverfahrens ist, dass 5s nach einer Erhöhung des Blutflusses ein Pegelsenken in der venösen Kammer durchgeführt wird. Mit der Erhöhung des Blutflusses wird eine Zeit gesetzt, bis zu der das Drucküberwachungsfenster inaktiv ist. Während des Pegelsenkens ist ein Bit der Bitmaske (in der Zustandstabelle T) gesetzt, das ebenfalls dazu führt, dass das Drucküberwachungsfenster deaktiviert wird. Die Drucküberwachungsfenster werden deaktiviert, wenn diese Bitmaske von "0" verschieden ist. Nach Abschluss des Pegelsenkens wird dieses Bit der Bitmaske zum Deaktivieren des Drucküberwachungsfensters zurückgenommen und die Zeit gesetzt, bis zu der das Drucküberwachungsfenster inaktiv ist. Dies ist dieselbe Variable, die auch mit der Erhöhung des Blutflusses gesetzt wurde.

In einer bevorzugten Ausführungsform der Erfindung wird eine CIassID verwendet. Dies ist eine Nummer, die für alle jeweils die korrespondierenden Ereignisse (z. B. Blutpumpenstopp und Blutpumpenstart) übereinstimmt. Die Reaktionen sind in Tabellen (sogenannten LookupTables) aufgelistet. Sie können auch in der Zustandstabelle T hinterlegt sein.

Soll die erfindungsgemäße Skalenend- bzw. erweiterte Skalenend-Überwachung auf ein druckrelevantes Ereignis reagieren, so muss das Flag ReactionRequired in der PressureReactionScaleEndLookupTable bzw. in der PressureReactionExtScaleEndLookupTable gesetzt sein.

Als Beispiel für eine zeitunabhängige Deaktivierung einer Grenze bzw. eines Grenzwertes GW (LowerLimitlnactive-, UpperLimitlnactive_) sei folgende Situation beschrieben: Soll eine Grenze für eine unbestimmte Zeit deaktiviert werden, muss das Flag LowerLimitlnactive_ bzw. UpperLimitlnactive_ gesetzt sein. Soll diese Deaktivierung zurückgenommen werden, muss dies bei einem korrespondierenden Ereignis mit derselben Classld_ geschehen, wobei dann die Varaiblen wie folgt gesetzt sind: ReactionRequired_ = 1 und LowerLimitlnactive_ bzw. UpperLimitlnactive_ = 0 sind. Beispiele für korrespondierende Ereignisse sind: SECURE_STATE_BLOOD_START und SECURE_STATE_BLOOD_END. Bei den beiden vorstehend aufgeführten Ereignissen handelt es sich jeweils um eine Beschreibung eines Zustandes einer Dialysemaschine. Dabei können insbesondere Aktoren dieser Maschine, wie unter anderem Pumpen und Klemmen, so geschaltet sein, dass von dieser Schaltung keine Gefahr für den Patienten ausgehen kann. In der Regel bedeutet dies: Pumpen gestoppt und Klemmen geschlossen.

Im Folgenden wird die zeitabhängige Deaktivierung einer Grenze (LowerLimitlnactiveDuration-, UpperLimitlnactiveDuration_) beschrieben. Soll eine Grenze für eine bestimmte Zeit deaktiviert werden, muss der Wert von LowerLimitlnactiveDuration_ bzw. UpperLimitlnactiveDuration_ auf den entsprechenden Wert in ms gesetzt sein. Nach Ablauf dieser Zeit ist dann die entsprechende Grenze wieder aktiv, wenn sie nicht durch andere Ereignisse verändert worden ist.

Erfindungsgemäß sind mehrere Steuerungen als Reaktionen der Fensterüberwachung auf druckrelevante Ereignisse vorgesehen. Soll die Fenster-Überwachung auf ein druckrelevantes Ereignis reagieren, so muss das Flag ReactionRequired_ in der PressureReactionWindowLookup-Table gesetzt sein.

Neben der vorstehend beschriebenen zeitabhängigen Grenzwertsetzung beziehen sich alternative Ausführungsformen der Erfindung auf eine permanente (zeitunabhängige) Deaktivierung einer Grenze (Inactive_). Soll eine Grenze für eine unbestimmte Zeit deaktiviert werden, muss das Flag Inactive_ gesetzt sein. Soll diese Deaktivierung zurückgenommen werden, muss dies bei einem korrespondierenden Ereignis mit derselben Classld_ geschehen, wobei dann die Variablen bevorzugt wie folgt gesetzt sind: ReactionRequired_ = 1 und Inactive_ = 0. Beispiele für korrespondierende Ereignisse sind: SECURE_STATE_BLOOD_START und SECURE_STATE_BLOOD_END.

Zeitabhängige Deaktivierung einer Grenze (InactiveDuration_):
Soll eine Grenze für eine bestimmte Zeitdauer deaktiviert werden, sollte der Wert von InactiveDuration_ auf den entsprechenden Wert in ms gesetzt sein. Nach Ablauf dieser Dauer ist die entsprechende Grenze wieder aktiv, wenn sie nicht durch andere Ereignisse bzw. im ADI - Tool deaktiviert wurde. Der Begriff ADI-Tool kennzeichnet ein elektronisches Modul, das als separates Gerät oder als Bestandteil einer Dialysemaschine, insbesondere des Monitors einer Dialysemaschine ausgebildet sein kann. Im ADI-Tool laufen alle Informationen zusammen, die zur Ausgabe von ereignisgesteuerten Meldungen auf dem Monitor der Dialysemaschine führen. Dabei sind auch die ereignisgesteuerten Reaktionen der Aktoren enthalten. Im ADI-Tool werden unabhängig von den auftretenden Ereignissen Programmzustände (z.B. Vorbereitung, Behandlung, Abrüsten) erkannt, die grundsätzlich Voraussetzung für die Ausführung der ereignisgesteuerten Reaktion sind.

Verlängerung überwachungsrelevanter Zeiten (ProlongActive_):
Soll die Zeitzählung für überwachungsrelevante Zeiten ausgesetzt werden (Verlängerung der überwachungsrelevanten Zeit), so wird vorzugsweise das Flag ProlongActive_ gesetzt. Ist dieses Flag gesetzt, so werden in jedem Programmdurchlauf folgende Zeiten um die Dauer eines Programm-Durchlaufs vergrößert (d.h., die Aktion wird um die Dauer nach hinten verschoben, für die das Flag gesetzt ist):
- Endzeitpunkt der Deaktivierung der Fensterüberwachung
- Startzeitpunkt zum Aufweiten des Fensters (untere und obere Grenze)
- Zeitpunkt zum Zurücksetzen des Fensters untere und obere Grenze)
- Zeitpunkt, bis zu dem das Fenster auf die Bereichsgrenze gesetzt werden soll (untere und obere Grenze)
- Zeitpunkt, bis zu dem das Verschieben des Fensters innerhalb der Bereichsgrenze erlaubt ist (nach unten und oben)
- Zeitpunkt zum automatischen Neusetzen des Fensters

Aufweitung einer Grenze - Allgemein (EnlargeStartDelay_, EnlargeTargetValue_):
Unter dem Begriff "Aufweitung einer Grenze" soll das Vergrößern des Grenzwertfensters verstanden werden, bei dem entweder der untere Grenzwert verkleinert oder der obere Grenzwert erhöht wird.

Ist der Wert der Variablen EnlargeStartDelay_ <> (ungleich) 0 gesetzt, wird die Aufweitung nach Ablauf dieser Zeitdauer (in ms) vorgenommen, und der neue Grenzwert ergibt sich aus dem dann vorliegenden Druck und dem in der Variablen EnlargeTargetValue_ stehenden Wert.

Ist der Wert der Variable EnlargeStartDelay =0 gesetzt, wird die Aufweitung sofort vorgenommen, und der neue Grenzwert ergibt sich aus dem alten Grenzwert und dem in der Variablen EnlargeTargetValue_ stehenden Wert.

Unabhängig davon, wie die Aufweitung vorgenommen wird, wird dies von dem System in einer Datenbank automatisch gespeichert. Insbesondere in den Variablen EnlargeDownValue_ bzw. EnlargeUpValue_ wird der Wert gespeichert, um den der ursprüngliche Grenzwert aufgeweitet wird.

Steht in der Variablen EnlargeValue_ ein Wert größer als 0, so wirkt die Aufweitung bevorzugt auf den oberen Grenzwert des Grenzwertfensters. Andernfalls, wenn also in der Variablen EnlargeValue_ ein Wert kleiner 0 gespeichert ist, so wirkt die Aufweitung bevorzugt auf den unteren Grenzwert des Grenzwertfensters.

Sollte der sich bei der Aufweitung ergebende neue Grenzwert innerhalb des "alten" Grenzwertfensters liegen, so bleibt das Grenzwertfenster in seiner ursprünglichen Ausdehnung erhalten.

Sollte zum Zeitpunkt einer Aufweitungsanforderung schon ein Aufweitungsvorgang ausgeführt werden, so wird das Fenster nur dann weiter aufgeweitet, wenn die neue Aufweitungsanforderung über die schon bestehende Aufweitung hinausgeht (d.h., eine neue Aufweitungsanforderung wird nicht zu einer schon bestehenden Aufweitungsanforderung hinzuaddiert).

Die Aufweitung wird dann zum Zeitpunkt zurückgenommen, wenn die letzte der überlappend stattfindenden Aufweitungen abläuft.

Mit dem automatischen Neusetzen eines Grenzwertfensters über die Funktion centerPressureWindow() wird eine evtl. aktive Aufweitung eines oder mehrerer Grenzwertfenster zurückgenommen und das Grenzwertfenster um den jeweils aktuellen Druck gesetzt.

Permanente Aufweitung einer Grenze (EnlargedFlag_):
Soll eine Grenze für eine unbestimmte Zeit aufgeweitet werden, sollte beispielsweise das Flag EnlargedFlag_ gesetzt sein.

Soll diese Aufweitung zurückgenommen werden, ist es vorzugsweise vorgesehen, dass dies bei einem korrespondierenden Ereignis mit derselben Classld_ ausgeführt wird, wobei die Variablen folgendermaßen gesetzt werden: ReactionRequired_ = 1 und EnlargedFlag_ = 0.

Zeit-oder volumenabhängige Aufweitung einer Grenze (EnlargeResetDelay_, EnlargeResetVolume_):
Soll eine Grenze für eine bestimmte Zeitdauer aufgeweitet werden, sollte z.B. der Wert von der Variablen EnlargeResetDelay_ auf den entsprechenden Wert in ms gesetzt sein.

Soll eine Grenze für ein bestimmtes (von der Blutpumpe zu förderndes) Volumen aufgeweitet werden, sollte beispielsweise der Wert von EnlargeResetVolume_ auf den entsprechenden Wert in ml gesetzt sein. Nach Ablauf dieser Zeit bzw. dieses Volumens ist dann die entsprechende Grenze wieder aktiv, wenn sie nicht durch andere Ereignisse deaktiviert wurde.

Nachführen des Grenzwertfensters innerhalb der Aufweitungsgrenzen (EnlargeMoveAllowed_):
Soll das Überwachungsfenster nach Ablauf der Aufweitung innerhalb der aufgeweiteten Fensterüberwachungsgrenzen dem Druck nachgeführt (verschoben) werden können, sollte z.B. das Flag EnlargeMoveAllowed_ gesetzt sein. Dann wird das Überwachungsfenster nach Ablauf der Aufweitung wieder so um den dann aktuellen Druck gesetzt, wie es um den Druck vor der Aufweitung gelegen hat. Sollte das Fenster außerhalb der Aufweitungsgrenzen zu liegen kommen, wird es beim Setzen nur so weit verschoben, wie es die aufgeweiteten Grenzen zulassen.

Nachführen des Grenzwertfensters (MoveUpAllowed_, MoveDownAllowed_):
Soll das Grenzwertfenster (z.B. bei sich ändernden Blutfluß) nur in eine Richtung mit dem sich dann ändernden Druck nachgeführt werden, werden bevorzugt das Flag MoveUpAllowed_ (Nachführen nach oben) bzw. MoveDownAllowed_ (Nachführen nach unten) sowie Inactive_bzw. InactiveDuration_ gesetzt. Mit der Anforderung wird sich die aktuelle Position des Drucks im Grenzwertfenster gespeichert und anschließend das (inaktive) Grenzwertfenster mit dem sich ändernden Druck nachgeführt (wenn sich der Druck in die erlaubte Richtung ändert). Mit Zurücknahme des Inactive_-Flags bzw. Ablauf der InactiveDuration_-Dauer wird das Grenzwertfenster auf den bis dahin nachgeführten aktuellen Stand gesetzt.

Neusetzen des Grenzwertfensters (WindowSetRequired_):
Der Begriff "Neusetzen" bedeutet, dass das Grenzwertfenster entsprechend der ursprünglichen Setup-Werte (zentriert oder asymmetrisch) bzw. die letzten aktiv vom Anwender des Systems eingestellten Werte (wenn der Anwender in der laufenden Behandlung das schon einmal gemacht hat) um den aktuellen Druck gesetzt wird.

Soll das Grenzwertfenster neu gesetzt werden, müssen vorzugsweise die Flags WindowSetRequired_ sowie Inactive_bzw. InactiveDuration_ gesetzt werden.

Mit Zurücknahme des Inactive_-Flags bzw. Ablauf der InactiveDuration_-Dauer wird das Grenzwertfenster um den dann aktuellen Druck neu gesetzt (wieder neu um den dann aktuellen Druck positioniert).

Permanentes Maximieren des Grenzwertfensters (Setzen auf Skalenenden - SetToBoundary_):
Soll eine Grenze für eine unbestimmte Zeit auf die Skalenenden gesetzt werden, muss z.B. das Flag SetToBoundary_ gesetzt sein. Soll das Maximieren zurückgenommen werden, sollte dies bei einem korrespondierenden Ereignis mit derselben Classld_ geschehen, wobei die Variablen wie folgt gesetzt sein können: ReactionRequired_ =1 und SetToBoundary_ =0. Das Grenzwertfenster wird dann auf die vor dem Maximieren aktiven Grenzen gesetzt.

Zeitabhängiges Maximieren des Grenzwertfensters (Setzen auf Skalenenden SetToBoundaryDuration_):
Soll eine Grenze für eine bestimmte Zeit auf die Skalenenden gesetzt werden, wird der Wert von der Variablen SetToBoundaryDuration_ vorzugsweise auf den entsprechenden Wert in ms gesetzt sein. Mit Ablauf der SetToBoundaryDuration_-Dauer wird das Grenzwertfenster auf die vor dem Maximieren aktiven Grenzen gesetzt.

Die Grenzwertsetzvorrichtung 200 kann einen elektrischen Controller (nicht dargestellt) umfassen, der einen integrierten Mikroprozessor aufweist und zur Ausführung des erfindungsgemäßen Verfahrens bestimmt ist. Der Controller kann vorzugweise als Steuergerät ausgebildet sein, wobei das Steuergerät dazu bestimmt ist, alle Schritte des Verfahrens zur ereignisgesteuerten Grenzwertsetzung im Rahmen der Geräteüberwachung auszuführen.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Für einen Fachmann ist es insbesondere offensichtlich, dass die Erfindung nicht nur für Hämodialysegeräte angewendet werden kann, sondern auch für andere medizintechnische Geräte, die auf Grenzwerteinhaltung überwacht werden müssen. Des weiteren können in der Zustandstabelle T noch weitere Anforderungen und Bedingungen codiert sein, die während der Überwachungsphase geprüft werden. Des weiteren können die Bauteile der Grenzwertsetzvorrichtung 200 auf mehrere physikalische Produkte verteilt realisiert werden kann.

### Bezugszeichenliste:

- 1000: Vorbestimmen
- 2000: Definieren
- 3000: Erfassen von Messsignalen
- 4000: Vergleich Messsignalvektor mit Referenz-Zustandsvektor
- 5000: Feststellen einer Übereinstimmung zwischen Messsignalvektor und Referenz-Zustandsvektor
- 6000: Steuerung des Gerätes mit Ziel-Grenzwerteinstellung
- 7000: Überwachung des Dialysegerätes

- 110: Dialysegerät
- 101: arterielle Blutleitung
- 102: Blutpumpe
- 103: Dialysator
- 104: venöse Blutleitung
- 105, 106: Dialysatleitungen

- ZV: Zustandsvektor
- R-ZV: Referenz-Zustandsvektor
- Z-GW: Ziel-Grenzwert
- T: Zustandstabelle

- 200: Grenzwertsetzvorrichtung
- 210: Bestimmungseinrichtung
- 220: Messsignalaufnehmer
- 230: Komparatorschaltung
- 240: Überwachungseinheit
- MEM: Speicher

- 250: Steuergerät
- UI: Ein- und Ausgabeschnittstelle

## Patentansprüche

1. Verfahren zur ereignisgesteuerten Grenzwertsetzung im Rahmen einer Geräteüberwachung auf Einhaltung von Grenzwerten in einem medizintechnischen Gerät (110), umfassend folgende Verfahrensschritte:
- Vorbestimmen (1000) einer Vielzahl von grenzwertrelevanten Ereignissen, die bei der Geräteüberwachung zu berücksichtigen sind und die jeweils als Zustandsvariable in einem Zustandsvektor (ZV) gespeichert werden
- Definieren (2000) von Referenz-Zustandsvektoren (R-ZV), denen jeweils eine Ziel-Grenzwerteinstellung (Z-GW) zugeordnet ist
- Erfassen von Messsignalen (3000) für alle oder ausgewählte Zustandsvariablen zur Berechnung eines Messsignalvektors
- Vergleich (4000) des berechneten Messsignalvektors mit den jeweils definierten Referenz-Zustandsvektoren (R-ZV) und bei Übereinstimmung (5000):
o Steuerung (6000) und Überwachung (7000) des medizintechnischen Gerätes (110) mit der Ziel-Grenzwerteinstellung (Z-GW), wobei jeweils ein grenzwertrelevantes Ereignis durch zumindest ein Bit einer Bitmaske repräsentiert und verarbeitet wird und wobei für die Einstellung jeweils eines Ziel-Grenzwertes (Z-GW) eine eigene Bitmaske verarbeitet wird.

2. Verfahren nach Patentanspruch 1, bei dem das medizintechnische Gerät (110) ein Gerät in einem extrakorporalen Flüssigkeitskreislaufsystem und insbesondere ein Blutbehandlungsgerät ist und insbesondere als Dialysegerät ausgebildet sein kann.

3. Verfahren nach einem der vorstehenden Patentansprüche, bei dem das Verfahren folgenden Verfahrensschritt umfasst:
- Bestimmen eines Zeitfensters, in dem die ereignisgesteuerte Grenzwertsetzung aktiviert oder deaktiviert sein soll.

4. Verfahren nach einem der vorstehenden Patentansprüche, bei dem eine Vielzahl von Referenz-Zustandsvektoren (R-ZV) mit der jeweils zugeordneten Ziel-Grenzwerteinstellung (Z-GW) als Zustandstabelle (T) mit zumindest zwei Zustandsvariablen gespeichert werden.

5. Verfahren nach einem der vorstehenden Patentansprüche, bei dem die Zustandsvariablen einer Zustandstabelle (T) binäre Zustandsvariablen umfassen und einen physikalischen Parameter des medizinischen Flüssigkeitskreislaufsystems betreffen.

6. Verfahren nach einem der vorstehenden Patentansprüche, bei dem die Einstellung der Ziel-Grenzwerte (Z-GW) eine Aufweitung oder eine Verkleinerung eines Grenzwertfensters, ein Nachführen eines unteren oder oberen Grenzwertes nach oben oder unten und eine Neueinstellung umfassen.

7. Verfahren nach einem der vorstehenden Patentansprüche, bei dem wechselseitige Abhängigkeiten zwischen den grenzwertrelevanten Ereignissen zur Einstellung eines Ziel-Grenzwertes (Z-GW) in einer Zustandstabelle (T) codiert sind.

8. Verfahren nach einem der vorstehenden Patentansprüche, bei dem die Geräteüberwachung eine Drucküberwachung in dem medizinischen Flüssigkeitskreislaufsystem umfasst und insbesondere eine Drucküberwachung eines Hämodialysegerätes (110) ist.

9. Verfahren nach einem der vorstehenden Patentansprüche, bei dem erfasste Messsignale immer im Verhältnis zum ursprünglichen Grenzwert gemessen und/oder gespeichert werden.

10. Verfahren nach einem der vorstehenden Verfahrensansprüche, bei dem das Verfahren mehrere Gerätezustände von mehreren Flüssigkeitskreislaufsystemabschnitten des Flüssigkeitskreislaufes parallel überwacht, wobei jeweils ein Ziel-Grenzwert (Z-GW) jeweils einem Flüssigkeitskreislaufsystemabschnitt zugeordnet ist.

11. Verfahren nach einem der vorstehenden Verfahrensansprüche, wobei bei einer Änderung eines Messsignals zumindest eines grenzwertrelevanten Ereignisses die Verfahrensschritte der Steuerung (6000) und der Überwachung (7000) rekursiv ausgeführt werden.

12. Grenzwertsetzvorrichtung (200) zur ereignisgesteuerten Grenzwertsetzung in einem medizintechnischen Gerät (110), mit:
- Einer Bestimmungseinrichtung (210), die zum Vorbestimmen von einer Vielzahl von grenzwertrelevanten Ereignissen ausgelegt ist, die bei der Geräteüberwachung zu berücksichtigen sind und die jeweils als Zustandsvariable in einem Zustandsvektor (ZV) gespeichert werden, wobei die Bestimmungseinrichtung (210) weiterhin zum Definieren von Referenz-Zustandsvektoren (R-ZV) bestimmt ist, denen jeweils eine spezifische Ziel-Grenzwerteinstellung (Z-GW) zugeordnet ist und wobei jeweils ein grenzwertrelevantes Ereignis durch zumindest ein Bit einer Bitmaske repräsentiert und verarbeitet wird
- Einem Messsignalaufnehmer (220) für jeweils einen Gerätezustand in dem medizinischen Flüssigkeitskreislaufsystem zum Erfassen von Messsignalen für alle oder ausgewählte der bestimmten grenzwertrelevanten Ereignisse zur Berechnung eines Messsignalvektors
- Einer Komparatorschaltung (230), die dazu bestimmt ist, den berechneten Messsignalvektor mit den jeweiligen definierten Referenz-Zustandsvektor (R-ZV) zu vergleichen und bei Übereinstimmung eine Überwachungseinheit (240) zu aktivieren
- Der Überwachungseinheit (240), die dazu bestimmt ist, die jeweilige dem Referenz-Zustandsvektor (R-ZV) zugeordnete Ziel-Grenzwerteinstellung (Z-GW) aus einem Speicher (MEM) auszulesen und das medizintechnische Gerät (110) auf Einhaltung der jeweils eingestellten Ziel-Grenzwerte (Z-GW) zu überwachen und wobei für die Einstellung jeweils eines Ziel-Grenzwertes (Z-GW) eine eigene Bitmaske verarbeitet wird
- Dem Speicher (MEM) zur Speicherung der eingestellten Ziel-Grenzwerte (Z-GW).

13. Grenzwertsetzvorrichtung (200) nach dem vorstehenden Vorrichtungsanspruch, wobei die Grenzwertsetzvorrichtung (200) in einen Geräteüberwachungsmonitor integriert oder an diesen als separate Vorrichtung angeschlossen ist, wobei der Geräteüberwachungsmonitor zur Geräteüberwachung auf Einhaltung von ereignisabhängigen Grenzwerten in dem medizintechnischen Gerät (110) bestimmt ist.

14. Grenzwertsetzvorrichtung (200) nach einem der vorstehenden Vorrichtungsansprüche, wobei das medizintechnische Gerät (110) ein extrakorporales Blutbehandlungssystem ist und eine Dialysatpumpe, eine Substituatspumpe und/oder eine Ultrafiltrationspumpe umfasst, deren Flüssigkeitsdruck jeweils ab- und/oder aufströmig der Pumpe gemessen und auf Einhaltung von Grenzwerten überwacht wird.

15. Grenzwertsetzvorrichtung (200) nach dem vorstehenden Vorrichtungsanspruch, wobei das extrakorporale Blutbehandlungssystem ein Dialysegerät (110) ist, das insbesondere zur Hämodialyse, zur Hämofiltration, zur Hämodiafiltration und/oder zur Peritonealdialyse eingerichtet ist.

16. Dialysegerät (110) mit einer Grenzwertsetzvorrichtung (200) nach einem der vorstehenden Vorrichtungsansprüche.

17. Medizintechnisches Gerät, insbesondere Dialysegerät (110), mit einem Steuergerät (250), wobei das Steuergerät (250) umfasst:
- Eine Ein- und Ausgangsschnittstelle (UI), die zum Einlesen von zumindest einem Zustandsvektor (ZV), der grenzwertrelevante Ereignisse als Kombination von Zustandsvariablen repräsentiert, und von zumindest einem Referenz-Zustandsvektor (R-ZV) mit einem Ziel-Grenzwert (Z-GW) bestimmt ist und wobei die Ein- und Ausgangsschnittstelle (UI) weiterhin zum Einlesen eines Messsignalvektors mit Messsignalen für alle oder ausgewählte Zustandsvariablen bestimmt ist, wobei jeweils ein grenzwertrelevantes Ereignis durch zumindest ein Bit einer Bitmaske repräsentiert und verarbeitet wird
- Eine Komparatorschaltung (230), die dazu bestimmt ist, den eingelesen Messsignalvektor mit dem jeweiligen Referenz-Zustandsvektor (R-ZV) zu vergleichen und bei Übereinstimmung eine Überwachungseinheit (240) zu aktivieren
- Der Überwachungseinheit (240), die dazu bestimmt ist, eine jeweils dem Referenz-Zustandsvektor (R-ZV) zugeordnete Ziel-Grenzwerteinstellung (Z-GW) aus einem Speicher (MEM) auszulesen und das medizintechnische Gerät (110) auf Einhaltung der jeweils eingestellten Ziel-Grenzwerte (Z-GW) zu überwachen, wobei für die Einstellung jeweils eines Ziel-Grenzwertes (Z-GW) eine eigene Bitmaske verarbeitet wird
- Dem Speicher (MEM), in dem zumindest die eingestellten Ziel-Grenzwerte (Z-GW) gespeichert ist.

## Claims

1. Method for event-controlled limit setting in the context of device monitoring for compliance with limit values in a medical technical (110), comprising the following method steps:
- Predetermining (1000) of a multitude of limit-relevant events to be taken into account during device monitoring, each of which is stored as a state variable in a state vector (ZV)
- Defining (2000) reference state vectors (R-ZV), each of which is assigned a target limit setting (Z-GW)
- Acquising measurement signals (3000) for all or selected state variables to calculate a measurement signal vector
- Comparison (4000) of the calculated measurement signal vector with the respective defined reference state vectors (R-ZV) and if they match (5000):
∘ Controlling (6000) and monitoring (7000) of the medical technical (110) with the target limit value setting (Z-GW), wherein in each case a limit value-relevant event is represented and processed by at least one bit of a bit mask and wherein a separate bit mask is processed for the setting in each case of a target limit value (Z-GW).

2. Method according to patent claim 1, in which the medical technical (110) is a device in an extracorporeal fluid circulation system and in particular a blood treatment device, and in particular can be designed as a dialysis device.

3. Method according to any one of the preceding claims, wherein the method comprises the following method step:
- Determining a time window in which the event-driven limit setting is to be activated or deactivated.

4. Method according to any one of the preceding claims, wherein a plurality of reference state vectors (R-ZV) each having an associated target limit setting (Z-GW) are stored as a state table (T) having at least two state variables.

5. Method according to any one of the preceding claims, wherein the state variables of a state table (T) comprise binary state variables and relate to a physical parameter of the medical fluid circulation system.

6. Method according to any one of the preceding claims, wherein setting target limit values (Z-GW) comprises widening or narrowing a limit value window, tracking a lower or upper limit value upward or downward, and readjusting.

7. Method according to any of the preceding claims, in which mutual dependencies between the events relevant to the limit value for setting a target limit value (Z-GW) are encoded in a state table (T).

8. Method according to any one of the preceding claims, wherein the device monitoring comprises pressure monitoring in the medical fluid circulation system, and in particular is pressure monitoring of a hemodialysis machine (110).

9. Method according to any of the preceding claims, in which detected measurement signals are always measured and/or stored in relation to the original limit value.

10. Method according to any one of the preceding method claims, wherein the method monitors a plurality of device states of a plurality of fluid circuit system sections of the fluid circuit in parallel, wherein a target limit value (Z-GW) is assigned to each fluid circuit system section.

11. Method according to one of the preceding method claims, wherein, in the event of a change in a measurement signal of at least one event relevant to the limit value, the method steps of the controller (6000) and of the monitor (7000) are executed recursively.

12. A limit setting device (200) for event-driven limit setting in a medical technical (110), comprising:
- A determination device (210) designed to predetermine a plurality of limit-relevant events to be taken into account in device monitoring, each of which is stored as a state variable in a state vector (ZV), wherein the determination device (210) is further designed to define reference state vectors (R-ZV), each of which is assigned a specific target limit setting (Z-GW), and wherein a limit-relevant event is represented and processed by at least one bit of a bit mask in each case
- A measurement signal transducer (220) for one device condition at a time in the medical fluid circulation system for acquiring measurement signals for all or selected ones of the determined limit-relevant events to calculate a measurement signal vector
- A comparator circuit (230) designed to compare the calculated measurement signal vector with the respective defined reference state vector (R-ZV) and to activate a monitoring unit (240) in the event of a match
- The monitoring unit (240), which is intended to read out the respective target limit value setting (Z-GW) assigned to the reference state vector (R-ZV) from a memory (MEM) and to monitor the medical technical (110) for compliance with the respectively set target limit values (Z-GW), and wherein a separate bit mask is processed for the setting of one target limit value (Z-GW) in each case
- The memory (MEM) for storing the set target limits (Z-GW).

13. Limit setting device (200) according to the preceding device claim, wherein the limit setting device (200) is integrated into a device monitoring monitor or is connected thereto as a separate device, wherein the device monitoring monitor is intended for device monitoring for compliance with event-dependent limit values in the medical technical (110).

14. Limit setting device (200) according to any one of the preceding device claims, wherein the medical technical (110) is an extracorporeal blood treatment system and comprises a dialysate pump, a substitute pump and/or an ultrafiltration pump, the fluid pressure of each of which is measured downstream and/or upstream of the pump and is monitored for compliance with limit values.

15. Limit setting device (200) according to the preceding device claim, wherein the extracorporeal blood treatment system is a dialysis device (110) which is set up in particular for hemodialysis, for hemofiltration, for hemodiafiltration and/or for peritoneal dialysis.

16. A dialysis machine (110) comprising a limit setting device (200) according to any one of the preceding device claims.

17. A medical technical, in particular dialysis device (110), with a control device (250), wherein the control device (250) comprises:
- An input and output interface (Ul) designed for reading in at least one state vector (ZV) representing limit-relevant events as a combination of state variables, and of at least one reference state vector (R-ZV) with a target limit value (Z-GW) and wherein the input and output interface (Ul) is further intended for reading in a measurement signal vector with measurement signals for all or selected state variables, wherein in each case a limit value-relevant event is represented by at least one bit of a bit mask and processed
- A comparator circuit (230) designed to compare the read-in measurement signal vector with the respective reference state vector (R-ZV) and to activate a monitoring unit (240) in the event of a match
- The monitoring unit (240), which is intended to read out a target limit value setting (Z-GW) assigned in each case to the reference state vector (R-ZV) from a memory (MEM) and to monitor the medical technical (110) for compliance with the target limit values (Z-GW) set in each case, a separate bit mask being processed for the setting of a target limit value (Z-GW) in each case
- The memory (MEM), in which at least the set target limit values (Z-GW) is stored.

## Revendications

1. Procédé de fixation de valeurs limites commandées par des événements dans le cadre d'une surveillance d'appareils pour le respect de valeurs limites dans un appareil médico-technique (110), comprenant les étapes de procédé suivantes :
- prédétermination (1000) d'une pluralité d'événements pertinents pour les valeurs limites, qui doivent être pris en compte lors de la surveillance de l'appareil et qui sont respectivement enregistrés comme variables d'état dans un vecteur d'état (VE)
- Définition (2000) de vecteurs d'état de référence (R-ZV), chacun étant associé à un réglage de valeur limite cible (Z-GW)
- Saisie de signaux de mesure (3000) pour toutes les variables d'état ou pour des variables d'état sélectionnées afin de calculer un vecteur de signaux de mesure
- comparaison (4000) du vecteur de signal de mesure calculé avec les vecteurs d'état de référence (R-ZV) définis respectivement et en cas de concordance (5000) :
∘ Commande (6000) et surveillance (7000) de l'appareil médico-technique (110) avec le réglage de la valeur limite cible (Z-GW), un événement pertinent pour la valeur limite étant respectivement représenté et traité par au moins un bit d'un masque de bit et un masque de bit propre étant traité pour le réglage respectivement d'une valeur limite cible (Z-GW).

2. Procédé selon la revendication 1, dans lequel l'appareil médico-technique (110) est un appareil dans un système de circulation de liquide extracorporel et en particulier un appareil de traitement du sang et peut être conçu en particulier comme un appareil de dialyse.

3. Procédé selon l'une quelconque des revendications précédentes, dans lequel le procédé comprend l'étape de procédé suivante :
- Déterminer une fenêtre de temps pendant laquelle la définition des limites en fonction des événements doit être activée ou désactivée.

4. Procédé selon l'une quelconque des revendications précédentes, dans lequel une pluralité de vecteurs d'état de référence (R-ZV) avec le réglage de valeur limite cible (Z-GW) respectivement associé sont stockés sous la forme d'un tableau d'état (T) avec au moins deux variables d'état.

5. Procédé selon l'une quelconque des revendications précédentes, dans lequel les variables d'état d'une table d'état (T) comprennent des variables d'état binaires et concernent un paramètre physique du système de circulation de fluide médical.

6. Procédé selon l'une quelconque des revendications précédentes, dans lequel le réglage des valeurs limites cibles (Z-GW) comprend un élargissement ou une réduction d'une fenêtre de valeurs limites, un suivi d'une valeur limite inférieure ou supérieure vers le haut ou vers le bas et un nouveau réglage.

7. Procédé selon l'une des revendications précédentes, dans lequel des dépendances mutuelles entre les événements pertinents pour la valeur limite sont codées dans un tableau d'état (T) pour le réglage d'une valeur limite cible (Z-GW).

8. Procédé selon l'une quelconque des revendications précédentes, dans lequel la surveillance de l'appareil comprend une surveillance de la pression dans le système de circulation de fluide médical et est en particulier une surveillance de la pression d'un appareil d'hémodialyse (110).

9. Procédé selon l'une quelconque des revendications précédentes, dans lequel les signaux de mesure détectés sont toujours mesurés et/ou mémorisés par rapport à la valeur limite initiale.

10. Procédé selon l'une des revendications de procédé précédentes, dans lequel le procédé surveille en parallèle plusieurs états d'appareils de plusieurs sections de système de circulation de liquide du circuit de liquide, une valeur limite cible (Z-GW) étant respectivement associée à une section de système de circulation de liquide.

11. Procédé selon l'une des revendications de procédé précédentes, dans lequel les étapes de procédé de la commande (6000) et de la surveillance (7000) sont exécutées de manière récursive lors d'une modification d'un signal de mesure d'au moins un événement pertinent pour la valeur limite.

12. Dispositif de fixation de valeur limites (200) pour la fixation de valeur limite commandée par événement dans un appareil médico-technique (110), comprenant :
- un dispositif de détermination (210), qui est conçu pour la prédétermination d'une pluralité d'événements pertinents pour les valeurs limites, qui doivent être pris en compte lors de la surveillance de l'appareil et qui sont respectivement mémorisés en tant que variable d'état dans un vecteur d'état (ZV), le dispositif de détermination (210) étant en outre destiné à définir des vecteurs d'état de référence (R-ZV), auxquels est respectivement associé un réglage de valeur limite cible spécifique (Z-GW) et un événement pertinent pour les valeurs limites étant respectivement représenté par au moins un bit d'un masque de bit et traité
- un capteur de signal de mesure (220) pour chaque état d'appareil dans le système de circulation de fluide médical pour l'acquisition de signaux de mesure pour tous les événements déterminés ou des événements sélectionnés parmi les événements pertinents pour la valeur limite pour le calcul d'un vecteur de signal de mesure
- un circuit comparateur (230), qui est destiné à comparer le vecteur de signal de mesure calculé avec le vecteur d'état de référence (R-ZV) défini respectif et à activer une unité de surveillance (240) en cas de concordance
- l'unité de surveillance (240), qui est destinée à lire dans une mémoire (MEM) le réglage de valeur limite cible (Z-GW) respectif associé au vecteur d'état de référence (R-ZV) et à surveiller l'appareil médico-technique (110) quant au respect des valeurs limites cibles (Z-GW) respectivement réglées et un masque binaire propre étant traité pour le réglage d'une valeur limite cible (Z-GW) respective
- la mémoire (MEM) pour enregistrer les valeurs limites cibles (VLC) réglées.

13. Dispositif de fixation de valeurs limites (200) selon la revendication de dispositif précédente, dans lequel le dispositif de fixation de valeurs limites (200) est intégré dans un moniteur de surveillance d'appareil ou est raccordé à celui-ci en tant que dispositif séparé, le moniteur de surveillance d'appareil étant destiné à la surveillance d'appareil pour le respect de valeurs limites dépendant d'événements dans l'appareil médico-technique (110).

14. Dispositif de fixation de valeurs limites (200) selon l'une des revendications de dispositif précédentes, dans lequel l'appareil médico-technique (110) est un système de traitement extracorporel du sang et comprend une pompe à dialysat, une pompe à substitut et/ou une pompe à ultrafiltration, dont la pression de liquide est mesurée respectivement en aval et/ou en amont de la pompe et est surveillée pour le respect de valeurs limites.

15. Dispositif de fixation de la valeur limite (200) selon la revendication précédente relative au dispositif, dans lequel le système de traitement extracorporel du sang est un appareil de dialyse (110), qui est notamment conçu pour l'hémodialyse, l'hémofiltration, l'hémodiafiltration et/ou la dialyse péritonéale.

16. Appareil de dialyse (110) avec un dispositif de fixation de valeur limites (200) selon l'une des revendications de dispositif précédentes.

17. Appareil médico-technique, en particulier appareil de dialyse (110), avec un appareil de commande (250), l'appareil de commande (250) comprenant:
- Une interface d'entrée et de sortie (UI), qui est destinée à lire au moins un vecteur d'état (ZV), qui représente des événements pertinents pour la valeur limite sous forme de combinaison de variables d'état, et d'au moins un vecteur d'état de référence (R-ZV) avec une valeur limite cible (Z-GW) et l'interface d'entrée et de sortie (UI) étant en outre destinée à la lecture d'un vecteur de signaux de mesure avec des signaux de mesure pour toutes les variables d'état ou pour des variables d'état sélectionnées, un événement pertinent pour la valeur limite étant respectivement représenté et traité par au moins un bit d'un masque de bits
- un circuit comparateur (230) qui est destiné à comparer le vecteur de signal de mesure lu avec le vecteur d'état de référence respectif (R-ZV) et à activer une unité de surveillance (240) en cas de concordance
- l'unité de surveillance (240), qui est destinée à lire dans une mémoire (MEM) un réglage de valeur limite cible (Z-GW) respectivement associé au vecteur d'état de référence (R-ZV) et à surveiller l'appareil médico-technique (110) quant au respect des valeurs limites cibles (Z-GW) respectivement réglées, un masque de bits propre étant traité pour le réglage d'une valeur limite cible (Z-GW) respective
- la mémoire (MEM), dans laquelle sont enregistrées au moins les valeurs limites cibles (VLC) réglées.
